# EUROPEAN PATENT APPLICATION

(11) **EP 4 600 265 A1**
(43) Date of publication of application: **13.08.2025**
(21) Application number: 23851311.3
(22) Date of filing: 23.05.2023
(51) Int. Cl.: C07K 14/605, C07K 1/02, C07K 1/06, C07C 309/66, C07C 303/30, C07C 237/22, C07C 231/12, C07D 209/56

(54) **PREPARATION METHOD FOR SEMAGLUTIDE, AND INTERMEDIATE**

(30) Priority: 09.08.2022 CN 202210947568
(71) Applicant: Yangzhou Aurisco Pharmaceutical Co., Ltd, Yangzhou, Jiangsu 225100 (CN); Aurisco Pharamceutical (Tianjin) Inc, Binhai New Area, Tianjin 300457 (CN)
(72) Inventor: GUO, Wancheng, Tianjin 300457 (CN); FANG, Jie, Tianjin 300457 (CN); DUAN, Yongli, Tianjin 300457 (CN); ZHANG, Fuchang, Tianjin 300457 (CN); WANG, Guoping, Yangzhou, Jiangsu 225100 (CN); YU, Zhenpeng, Yangzhou, Jiangsu 225100 (CN); YANG, Yingrui, Yangzhou, Jiangsu 225100 (CN)
(74) Representative: Dehns
(86) International application number: PCT/CN2023/095654
(87) International publication number: WO 2024/032081

(57) **Abstract**

The present invention provides a preparation method for semaglutide, and an intermediate. The preparation method comprises the steps of: (l) reacting compound SEM110 and compound SEM120 in a solvent under an alkaline condition to obtain compound SEM130; and (2) deprotecting the compound SEM130 to form semaglutide, the reaction formula being as follows. The preparation method of the present invention is simple to operate, has mild reaction conditions, is easy to purify, has high product purity and low cost, and is suitable for industrial production.

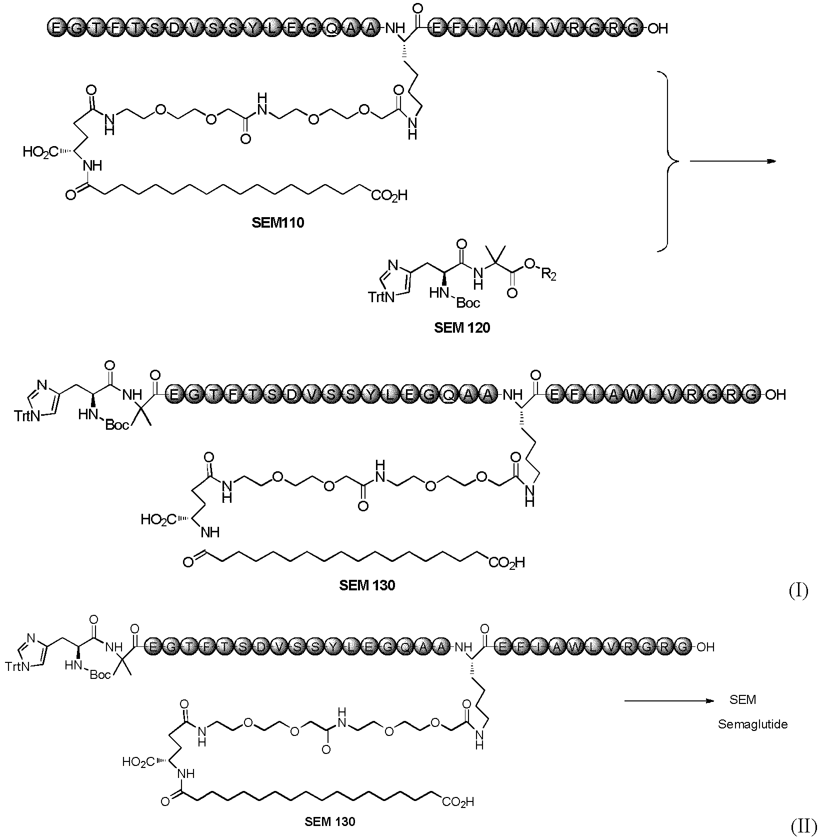

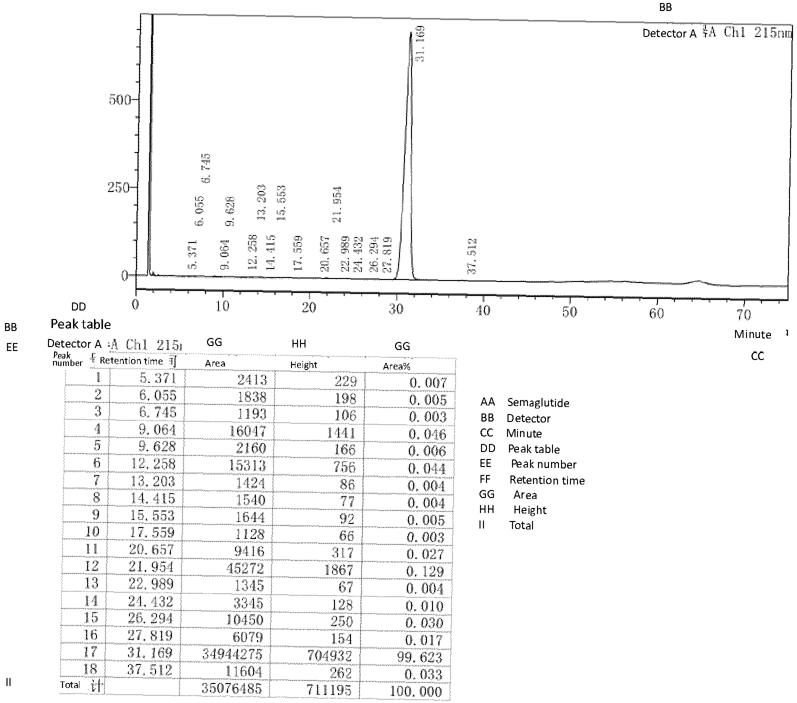

## Description

### FIELD OF THE INVENTION

The present invention relates to the field of drug preparation, and more specifically, to a preparation method for Semaglutide, and intermediate.

### BACKGROUND TECHNOLOGY

Diabetes mellitus is a common and frequently occurring endocrine and metabolic disease characterized by hyperglycemia and other complications caused by absolute or relative deficiency of insulin. China has the heaviest burden of diabetes, with more than 114 million people suffering from it. Glucagon-like peptide-1 (GLP-1) is an important incretin, and compared with traditional diabetes drugs, it can solve the problem of β-cell apoptosis that cannot be solved by conventional oral hypoglycemic drugs. Semaglutide is known as the world's best GLP-1 agonist, with 94% homology to human GLP-1, showing greater advantages in lowering blood sugar, losing weight, benefiting cardiovascular system, and safety. The lysine position at position 26 of the Semaglutide peptide chain is connected to an 18 carbon fatty diacid side chain, which can mediate and promote strong binding between Semaglutide and albumin, and reduce renal clearance rate. Compared with the liraglutide with C16 side chain, the affinity of Semaglutide side chain for albumin is enhanced by 5-6 times. Binding with albumin can increase the molecular weight of this product, avoid rapid clearance by kidney, prevent metabolic degradation, and prolong the half-life *in vivo* to achieve long-term effect. The structure of Semaglutide is as follows:

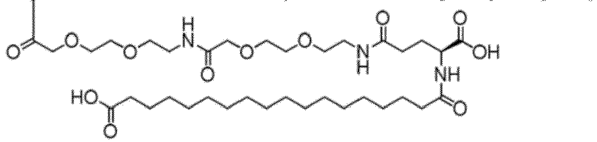

His⁷-Aib⁸-Glu⁹-Gly¹⁰-Thr¹¹-Phe¹²-Thr¹³-Ser¹⁴-Asp¹⁵-Val¹⁶-Ser¹⁷-Ser¹⁸-Tyr¹⁹-Leu²⁰-Glu²¹-Gly²²-
Gln²³-Ala²⁴-Ala²⁵-Lys²⁶-Glu²⁷-Phe²⁸-Ile²⁹-Ala³⁰-Trp³¹-Leu³²-Val³³-Arg³⁴-Gly³⁵-Arg³⁶-Gly³⁷

At present, the main methods for preparing Semaglutide are chemical and biological methods. Semaglutide is a medium length peptide containing 31 amino acid residues. If standard chemical methods are used, the process steps are multiple, the cycle is long, and the resin shrinks severely during gradual coupling, resulting in incomplete reaction and easy production of defective peptides, leading to high impurities in the product and difficulty in purification. If the solid-phase fragment condensation method is used, a large and excess amount of each fragment is required, while there are many side reactions, and product separation is difficult. In short, chemical methods have low yield, high cost, and require a large amount of organic solvent, making them unsuitable for industrial production. A typical biological method is the method disclosed in WO 2009083549A1, which uses gene recombination technology to produce the main peptide chain of Semaglutide using yeast, modifies the main peptide chain with side chain, and then connects the remaining short peptide. The end of the peptide chain of Semaglutide has a dipeptide His-Aib, and Aib is a non-natural amino acid. Semaglutide is usually obtained by expressing the main peptide Arg34GLP-1 (9-37) by recombinant fermentation, and then modifying side chain at ε-amino of Lys20 and connecting the dipeptide His-Aib by chemical synthesis. This method has short steps and the purification of the product is relatively simple and easy compared to chemical methods.

In the method of modifying the side chain of the main chain of Semaglutide and connecting short peptide disclosed in WO2009083549A1, both the side chain and short peptide are either activated using HOSU to obtain active esters and then coupled, or inactivated. The inventor of this application found in practical research that when using HOSU to prepare -OSU active esters of the side chain and the short peptide, one more molecules of amino propionic acid impurities (IMP1, IMP2) will be generated respectively. The derivative impurities of these two impurities have similar properties to the product, making it difficult to separate and purify, resulting in a large consumption of solvent, a high separation cost, and an unfavorable quality control in the production process. The structures of IMP1 and IMP2 are shown as follows:

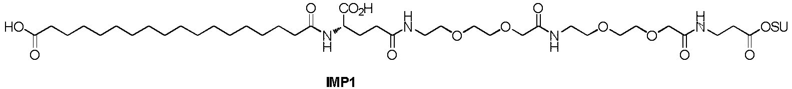

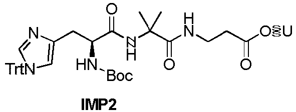

If inactivated side chains and short peptide are used to condense with Arg34GLP-1 (9-37), or Arg34GLP-1 (11-37), highly active condensation reagents are required. Arg34GLP-1 (9-37) or Arg34GLP-1 (11-37) itself contains multiple free carboxyl groups, and the carboxyl groups of the side chain and the short peptide condense competitively with the amino groups of the long peptide, resulting in poor selectivity of the target product and abnormally complex products.

Therefore, there is still an urgent need in this field to develop a new method for preparing Semaglutide with better impurity control and lower cost.

### SUMMARY OF THE INVENTION

In view of the disadvantages of low yield, high production cost, multiple waste liquid generated, and difficult product purification in the existing synthesis methods of semaglutide, the purpose of one aspect of the present invention is to provide a fragment condensation method for preparing Semaglutide, which can obtain Semaglutide product with high purity at low cost and high efficiency. To achieve the purpose of the present invention, the following technical solutions are adopted:

A preparation method for Semaglutide, comprising the steps of:
(1) reacting compound SEM110 and compound SEM120 in a solvent under an alkaline condition to obtain compound SEM130, and the reaction formula is as follows: 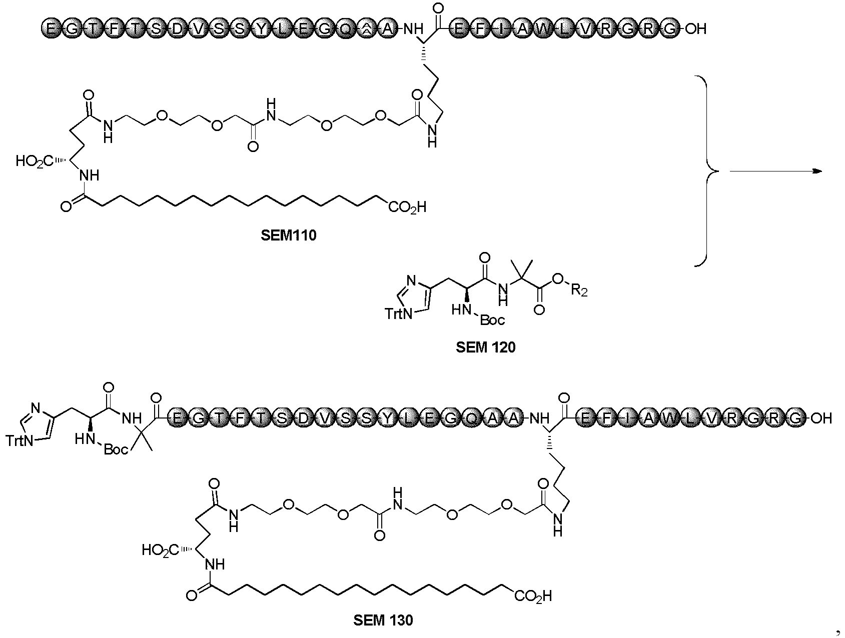 wherein, R₂ is *p*-nitrophenyl, methylsulfonyl, pentafluorophenyl or 5-norbornene-2,3-dicarboxamido;
(2) deprotecting the compound SEM130 to form Semaglutide, and the reaction formula is as follows: 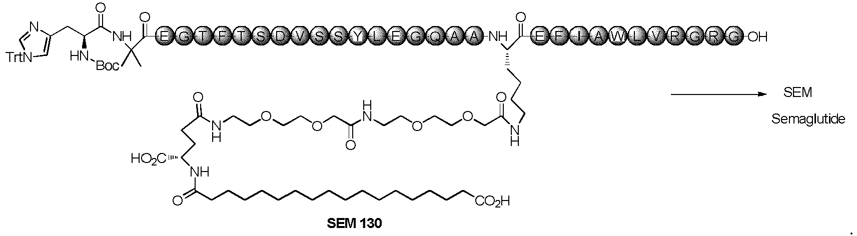
more preferably, R₂ is pentafluorophenyl.

Preferably, in step (1), the solvent is selected from one or more combinations of N, N-dimethylformamide, N-methylpyrrolidone, N, N-dimethylacetamide, tetrahydrofuran, dichloromethane, acetone, acetonitrile, and water.

Preferably, in step (1), the alkali is selected from one or more combinations of carbonates, phosphates, bicarbonates, hydrophosphates, triethylamine, DBU, DMAP, and diisopropylethylamine, and more preferably diisopropylethylamine.

Preferably, in step (1), the molar ratio of compound SEM110 to compound SEM120 is 1:1.0-4.0, more preferably 1:2.0-3.0, and most preferably 1:2.5.

Preferably, in step (1), the amount of alkali used is to maintain the pH of the system at 9-10, and more preferably 8-9.

Preferably, in step (2), the solvent used for the deprotection reaction is selected from one or more combinations of dichloromethane, methyl *tert*-butyl ether, trifluoroacetic acid, TIS, acetonitrile, and water.

Preferably, in step (2), the deprotection is carried out in the presence of acid.

Preferably, the acid is selected from one or more combinations of TFA, acetic acid, hydrochloric acid, hydrobromic acid, phosphoric acid, and methylsulfonic acid, and more preferably TFA, acetic acid.

Preferably, the volume to weight ratio of the acid to compound SEM130 is 1-10 mL/g.

Preferably, compound SEM120 is prepared by the following steps:
(1-d) reacting the compound shown in SEM115 and compound 2 in a solvent in the presence of a dehydrating agent to form SEM120, and the reaction formula is as follows: 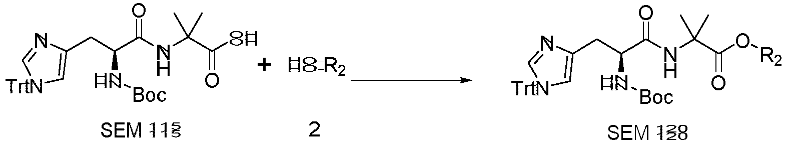
wherein, R₂ is *p*-nitrophenyl, methylsulfonyl, pentafluorophenyl or 5-norbornene-2,3-dicarboxamido.

Preferably, in step (1-d), the solvent is selected from one or more combinations of N, N-dimethylformamide, N-methylpyrrolidone, N, N-dimethylacetamide, tetrahydrofuran, dichloromethane, acetone, acetonitrile, and water.

Preferably, in step (1-d), the dehydrating agent is selected from one or more combinations of DCC, DIC, EDCI, and T₃P, and more preferably DCC.

Preferably, in step (1-d), the molar ratio of compound SEM115 to compound 2 is 1:1.0-1.4, more preferably 1:1.0-1.2, and most preferably 1:1.05.

Preferably, in step (1-d), the molar ratio of dehydrating agent to compound SEM115 is 1.4-1.0:1, more preferably 1.3-1.1:1, and most preferably 1.2:1.

Preferably, compound SEM110 is prepared by the following steps:
(1-c) reacting compound SEM100 and compound SEM105 in a solvent in the presence of an alkali to form SEM110, and the reaction formula is as follows: 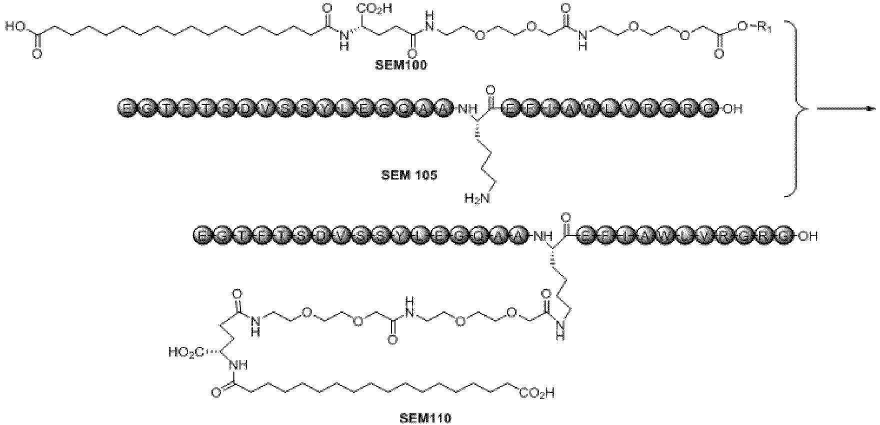
wherein, R₁ is *p*-nitrophenyl, methylsulfonyl, pentafluorophenyl or 5-norbornene-2,3-dicarboxamido.

More preferably, R₁ is 5-norbornene-2,3-dicarboxamido.

Preferably, in step (1-c), the solvent is selected from one or more combinations of N, N-dimethylformamide, N-methylpyrrolidone, N, N-dimethylacetamide, tetrahydrofuran, dichloromethane, acetone, acetonitrile, and water.

Preferably, in step (1-c), the alkali is selected from one or more combinations of carbonates, phosphates, bicarbonates, hydrophosphates, triethylamine, DBU, DMAP, and diisopropylethylamine, and more preferably diisopropylethylamine.

Preferably, in step (1-c), the molar ratio of compound SEM100 to compound SEM105 is 1.1-1.6:1, more preferably 1.2-1.5:1.

Preferably, in step (1-c), the amount of alkali used is in a molar ratio of 5-25:1 to compound SEM105, more preferably 10-20:1.

Preferably, compound SEM100 is prepared by the following steps:
(1-a) reacting compound SEM80 and compound 1 in a solvent in the presence of a dehydrating agent to form compound SEM90, and the reaction formula is as follows: 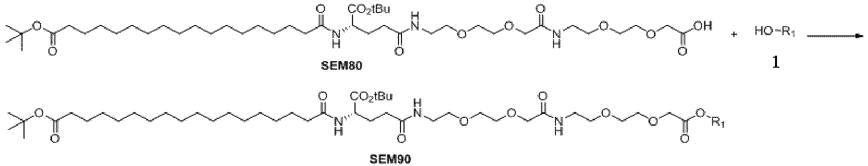 wherein, R₁ is *p*-nitrophenyl, methylsulfonyl, pentafluorophenyl or 5-norbornene-2,3-dicarboxamido;
(1-b) deprotecting the compound SEM90 in a solvent in the presence of acid to form compound SEM100, and the reaction formula is as follows: 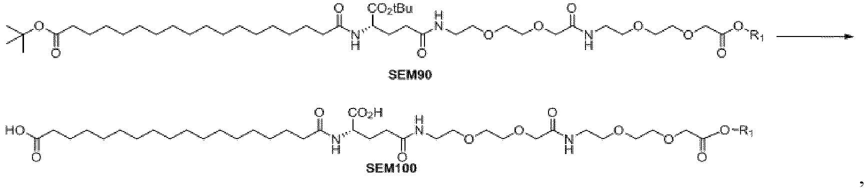 wherein, R₁ is *p*-nitrophenyl, methylsulfonyl, pentafluorophenyl or 5-norbornene-2,3-dicarboxamido.

Preferably, the solvent in step (1-a) is selected from one or more combinations of N, N-dimethylformamide, N-methylpyrrolidone, N, N-dimethylacetamide, tetrahydrofuran, dichloromethane, acetone, acetonitrile, and water.

Preferably, the dehydrating agent in step (1-a) is selected from one or more combinations of DCC, DIC, EDCI, and T₃P, more preferably DCC.

Preferably, in step (1-a), the molar ratio of compound SEM80 to compound 1 is 1:1.0-1.4, more preferably 1:1.0-1.2, and most preferably 1:1.05.

Preferably, in step (1-a), the molar ratio of compound SEM80 to the dehydrating agent is 1:1.0-1.4, more preferably 1:1.1-1.2.

Preferably, the solvent in step (1-b) is selected from one or more combinations of dichloromethane, trifluoroacetic acid, TIS, acetonitrile, and water.

Preferably, in step (1-b), the acid is selected from one or more combinations of trifluoroacetic acid, acetic acid, hydrochloric acid, hydrobromic acid, phosphoric acid, and methylsulfonic acid, more preferably trifluoroacetic acid, acetic acid.

Preferably, in step (1-b), the volume to weight ratio of the acid to compound 90 is 1-10 mL/g, more preferably 3-5 mL/g.

Another purpose of the present invention is to provide a compound SEM90 or salts thereof, wherein the structural formula of the compound is as follows: 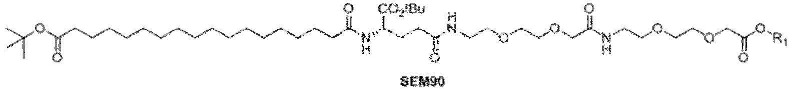 wherein, R₁ is methylsulfonyl or 5-norbornene-2,3-dicarboxamido.

Another purpose of the present invention is to provide a preparation method for compound SEM90, which comprises the following steps:
(1-a) reacting compound SEM80 and compound 1 in a solvent in the presence of a dehydrating agent to form SEM90, and the reaction formula is as follows: 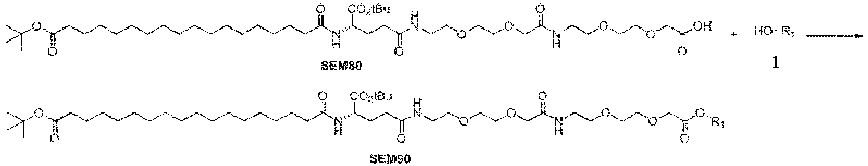
wherein, R₁ is methylsulfonyl or 5-norbornene-2,3-dicarboxamido;

Preferably, the solvent in step (1-a) is selected from one or more combinations of N, N-dimethylformamide, N-methylpyrrolidone, N, N-dimethylacetamide, tetrahydrofuran, dichloromethane, acetone, acetonitrile, and water.

Preferably, the dehydrating agent in step (1-a) is selected from one or more combinations of DCC, DIC, EDCI, and T₃P, more preferably DCC.

Preferably, in step (1-a), the molar ratio of compound SEM80 to compound 1 is 1:1.0-1.4, more preferably 1:1.0-1.2, and most preferably 1:1.05.

Preferably, in step (1-a), the molar ratio of compound SEM80 to the dehydrating agent is 1:1.0-1.4, more preferably 1:1.1-1.2.

Another purpose of the present invention is to provide a compound SEM100 or salts thereof, wherein the structural formula of the compound is as follows: 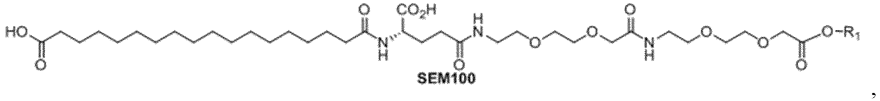 wherein, R₁ is methylsulfonyl, pentafluorophenyl or 5-norbornene-2,3-dicarboxamido.

Another purpose of the present invention is to provide a preparation method for compound SEM100, which comprises the following steps:
(1-b) deprotecting the compound SEM90 in a solvent in the presence of acid to form compound SEM100, and the reaction formula is as follows: 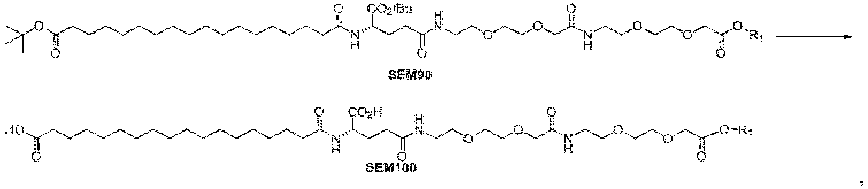
wherein, R₁ is methylsulfonyl, pentafluorophenyl or 5-norbornene-2,3-dicarboxamido.

Preferably, the solvent in step (1-b) is selected from one or more combinations of dichloromethane, trifluoroacetic acid, TIS, acetonitrile, and water.

Preferably, in step (1-b), the acid is selected from one or more combinations of trifluoroacetic acid, acetic acid, hydrochloric acid, hydrobromic acid, phosphoric acid, and methylsulfonic acid, more preferably trifluoroacetic acid, acetic acid.

Preferably, in step (1-b), the volume to weight ratio of the acid to compound 90 is 1-10 mL/g, more preferably 3-5 mL/g.

Another purpose of the present invention is to provide a compound SEM120 or salts thereof, wherein the structural formula of the compound is as follows: 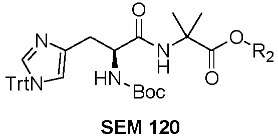 wherein, R₂ is methylsulfonyl, pentafluorophenyl or 5-norbornene-2,3-dicarboxamido.

Another purpose of the present invention is to provide a preparation method for an intermediate of the side chain of Semaglutide, which comprises the following steps:
(1-a) reacting compound SEM80 and compound 1 in a solvent in the presence of a dehydrating agent to form SEM90, and the reaction formula is as follows: 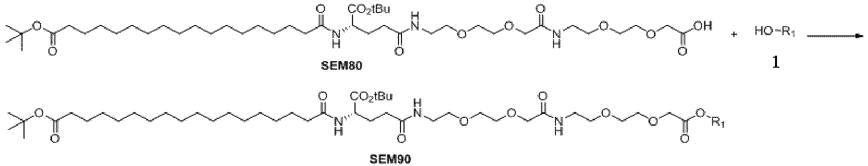
wherein, R₁ is *p*-nitrophenyl, methylsulfonyl, pentafluorophenyl or 5-norbornene-2,3-dicarboxamido

More preferably, R₁ is 5-norbornene-2,3-dicarboxamido.

Another purpose of the present invention is to provide a preparation method for the side chain of Semaglutide, which comprises the following steps:
(1-a) reacting compound SEM80 and compound 1 in a solvent in the presence of a dehydrating agent to form SEM90, and the reaction formula is as follows: 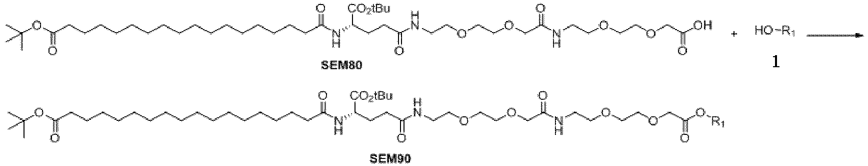 wherein, R₁ is *p*-nitrophenyl, methylsulfonyl, pentafluorophenyl or 5-norbornene-2,3-dicarboxamido;
(1-b) deprotecting the compound SEM90 in a solvent in the presence of acid to form compound SEM100, and the reaction formula is as follows: 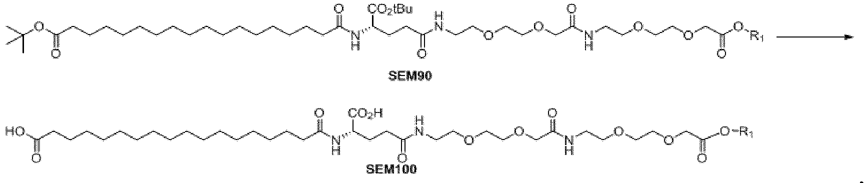 wherein, R₁ is *p*-nitrophenyl, methylsulfonyl, pentafluorophenyl or 5-norbornene-2,3-dicarboxamido.

More preferably, R₁ is 5-norbornene-2,3-dicarboxamido.

Another purpose of the present invention is to provide a preparation method for an intermediate of Semaglutide, which comprises the following steps:
(1-a) reacting compound SEM80 and compound 1 in a solvent in the presence of a dehydrating agent to form compound SEM90, and the reaction formula is as follows: 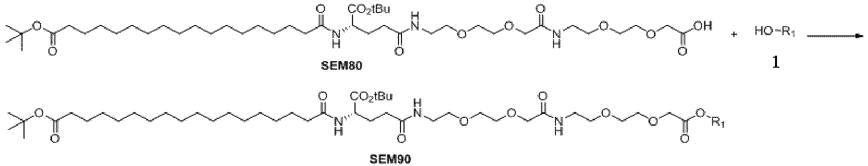 wherein, R₁ is *p*-nitrophenyl, methylsulfonyl, pentafluorophenyl or 5-norbornene-2,3-dicarboxamido;
(1-b) deprotecting the compound SEM90 in a solvent in the presence of acid to form compound SEM100, and the reaction formula is as follows: 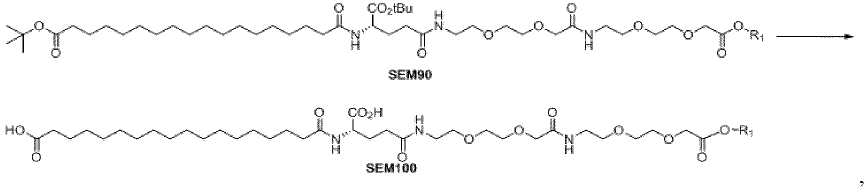 wherein, R₁ is *p*-nitrophenyl, methylsulfonyl, pentafluorophenyl or 5-norbornene-2,3-dicarboxamido;
(1-c) reacting compound SEM100 and compound SEM105 in a solvent in the presence of an alkali to form compound SEM110, and the reaction formula is as follows: 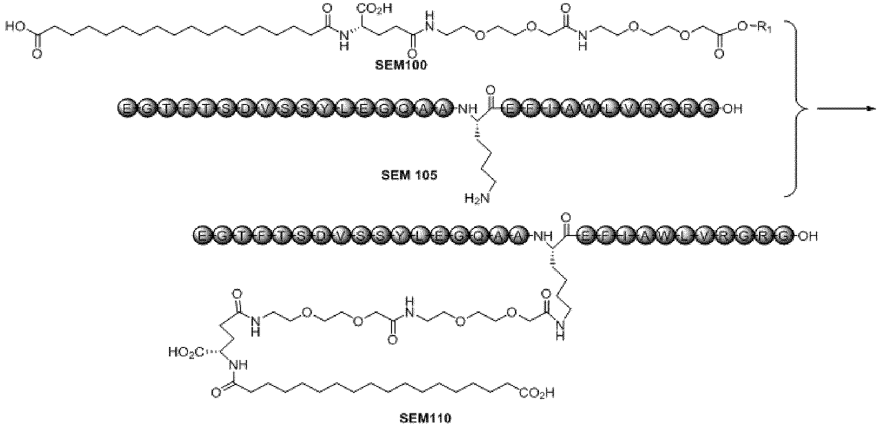 wherein, R₁ is *p*-nitrophenyl, methylsulfonyl, pentafluorophenyl or 5-norbornene-2,3-dicarboxamido.

More preferably, R₁ is 5-norbornene-2,3-dicarboxamido.

Another purpose of the present invention is to provide a preparation method for Semaglutide, comprising the following steps:
(1) reacting compound SEM110 and compound SEM120 in a solvent under an alkaline condition to obtain compound SEM130; and
(2) deprotecting the compound SEM130 to form Semaglutide,
   wherein, step (1) further includes the following steps:
      (1-a) reacting compound SEM80 and compound 1 in a solvent in the presence of a dehydrating agent to form SEM90;
      (1-b) deprotecting the compound SEM90 in a solvent in the presence of acid to form compound SEM100;
      (1-c) reacting the compound SEM100 and compound SEM105 in a solvent in the presence of an alkali to form compound SEM110;
      (1-d) reacting compound SEM115 and compound 2 in a solvent in the presence of a dehydrating agent to form SEM120;
   the reaction formula for the above steps is as follows: 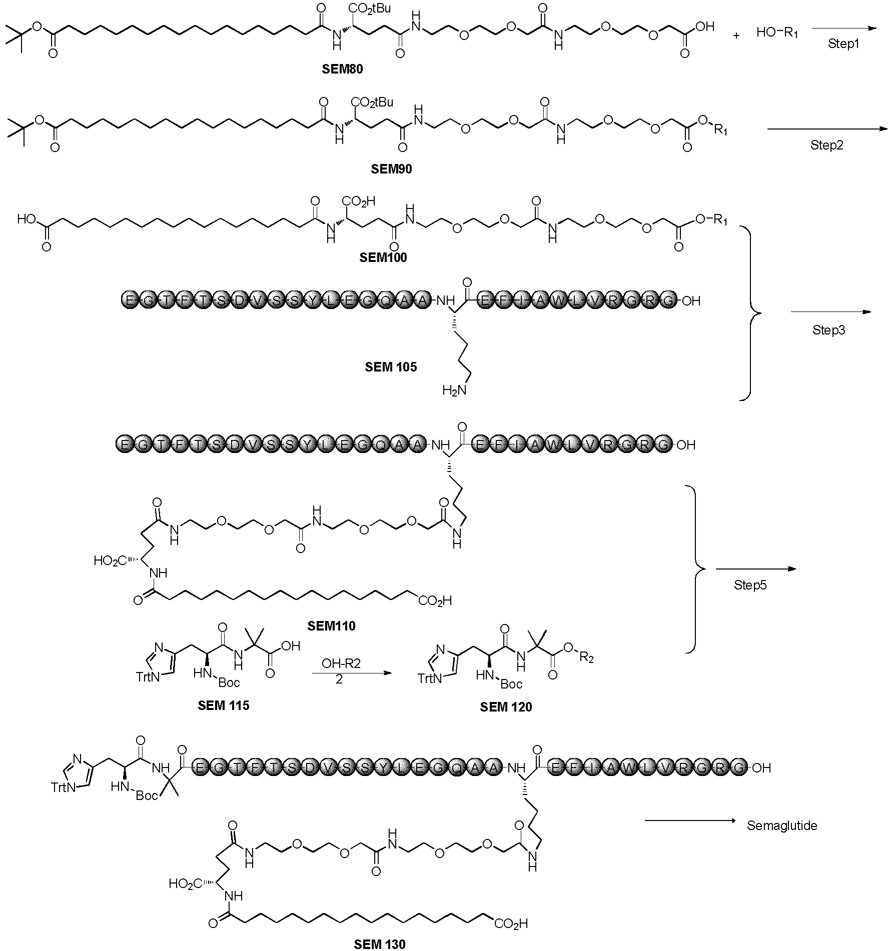
   wherein, R₁ is *p*-nitrophenyl, methylsulfonyl, pentafluorophenyl or 5-norbornene-2,3-dicarboxamido, R₂ is *p*-nitrophenyl, methylsulfonyl, pentafluorophenyl or 5-norbornene-2,3-dicarboxamido, and
   there is no order between steps (1-d) and steps (1-a) to (1-c), and they can be interchanged with any of the steps.

More preferably, R₁ is 5-norbomene-2,3-dicarbodiimide, and R₂ is pentafluorophenyl.

### DESCRIPTION OF THE DRAWINGS

Figure 1 is the HPLC chromatogram of compound SEM100A prepared in Example 1.
Figure 2 is the HPLC chromatogram of compound SEM100B prepared in Example 2.
Figure 3 is the HPLC chromatogram of compound SEM120A prepared in Example 3.
Figure 4 is the HPLC chromatogram of compound SEM120B prepared in Example 4.
Figure 5 is the HPLC chromatogram of compound SEM120C prepared in Example 5.
Figure 6 is the typical mass spectrum of compound SEM110.
Figure 7 is the HPLC chromatogram of compound SEM110 prepared in Example 6
Figure 8 is the HPLC chromatogram of compound SEM130 prepared in Example 6.
Figure 9 is the typical mass spectrum of compound SEM130.
Figure 10 is the HPLC chromatogram of compound SEM110 prepared in Example 7.
Figure 11 is the HPLC chromatogram of compound SEM130 prepared in Example 7.
Figure 12 is the HPLC chromatogram of compound SEM110 prepared in Example 8.
Figure 13 is the HPLC chromatogram of compound SEM130 prepared in Example 8.
Figure 14 is the HPLC chromatogram of compound SEM120D prepared in Example 9.
Figure 15 is the HPLC chromatogram of compound SEM110 prepared in Example 10.
Figure 16 is the HPLC chromatogram of compound SEM130 prepared in Example 10.
Figure 17 is the HPLC chromatogram of the solid crude product of Semaglutide prepared in Example 11 (with a total monitoring time of 40 minutes).
Figure 18 is the HPLC chromatogram of the refined and isolated Semaglutide prepared in Example 11 (with a total monitoring time of 75 minutes).
Figure 19 is the typical mass spectrum of Semaglutide (SEM).
Figure 20 is the HPLC chromatogram of the refined and isolated Semaglutide prepared in Example 12.
Figure 21 is the HPLC chromatogram of the refined and isolated Semaglutide prepared in Example 13.
Figure 22 is the HPLC chromatogram of the refined and isolated Semaglutide prepared in Example 14.
Figure 23 is the HPLC chromatogram of compound SEM100C prepared in Example 15.
Figure 24 is the typical mass spectra of compounds SEM100A, SEM100B, SEM100C, and SEM100D after being quenched with excess cyclohexylamine.
Figure 25 is the typical mass spectra of compounds SEM120A, SEM120B, SEM120C, and SEM120D after being quenched with excess cyclohexylamine.
Figure 26 is the HPLC chromatogram of compound SEM100D prepared in Example 16.
Figure 27 is the HPLC chromatogram of compound SEM130 prepared in Example 17.
Figure 28 is the HPLC chromatogram of compound SEM130 prepared in Example 18.
Figure 29 is the HPLC chromatogram of compound SEM130 prepared in Example 19.
Figure 30 is the HPLC chromatogram of Semaglutide prepared in Example 20.
Figure 31 is the HPLC chromatogram of Semaglutide prepared in Example 21.
Figure 32 is the HPLC chromatogram of Semaglutide prepared in Example 22.
Figure 33 is the HPLC chromatogram of compound SEM100E prepared in Comparative Example 1.
Figure 34 is the mass spectrum of the system containing IMP1 and SEM100E prepared in Comparative Example 1 after being quenched with cyclohexylamine.
Figure 35 is the HPLC chromatogram of compound SEM120E prepared in Comparative Example 2.
Figure 36 is the mass spectrum of the system containing IMP2 and SEM120E prepared in Comparative Example 2 after being quenched with water.
Figure 37 is the HPLC chromatogram of the refined and isolated Semaglutide prepared in Comparative Example 3.
Figure 38 is the HPLC chromatogram of the refined and isolated Semaglutide prepared in Comparative Example 4.
Figure 39 is the HPLC chromatogram of the refined and isolated Semaglutide prepared in Comparative Example 5.

### DETAILED DESCRIPTION OF THE INVENTION

In view of the defects in the preparation of Semaglutide in the prior art (patent publication number WO2009083549A1), the inventor of the present application conducted in-depth research and found that by replacing the existing active ester donor HOSU with nitrophenol ester, methanesulfonate, pentafluorophenol ester or HONB ester during the modification of the side chain and short peptide (dipeptide), impurities IMP1 and IMP2 are completely avoided, resulting in higher purity and higher yield of the final product Semaglutide. On this basis, the present invention has been completed.

In a preferred embodiment of the present invention, the preparation method of Semaglutide comprises the steps of: (1) reacting compound SEM110 and compound SEM120 in a solvent under an alkaline condition to obtain compound SEM130, and the reaction formula is as follows: 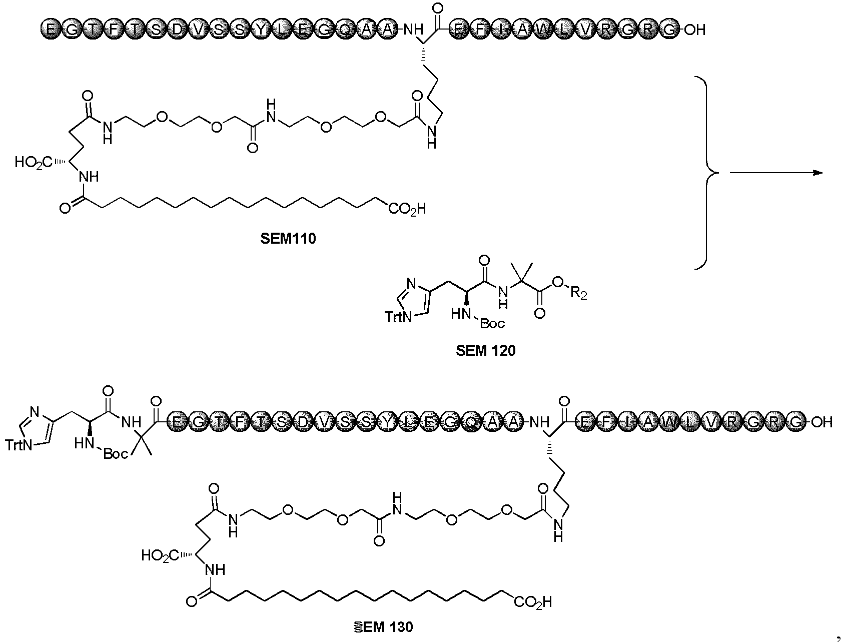
wherein, R₂ is *p*-nitrophenyl, methylsulfonyl, pentafluorophenyl or 5-norbornene-2,3-dicarboxamido;
(2) deprotecting the compound SEM130 to form Semaglutide, and the reaction formula is as follows:

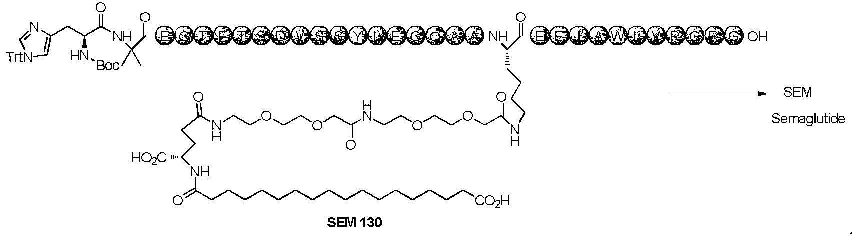

In step (1), the solvent used is a good solvent for all raw materials (compound SEM110 and compound SEM120), and the amount of solvent used is such that all raw materials are completely dissolved in it. The preferred solvent is one or more combinations of N, N-dimethylformamide, N-methylpyrrolidone, N, N-dimethylacetamide, tetrahydrofuran, dichloromethane, acetone, acetonitrile, and water. The preferred alkali used in step (1) is one or more combinations of carbonates, phosphates, bicarbonates, hydrophosphates, triethylamine, DBU, DMAP, and diisopropylethylamine, and more preferably diisopropylethylamine. The molar ratio of compound SEM110 to compound SEM120 is 1:1.0-4.0, and more preferably 1:2.0-3.0. The preferred amount of alkali is such that the pH of the reaction solution is 8-10, and more preferably 9-10. In step (1), the preferred reaction temperature is 0-25 °C, and more preferably 0-15 °C. Step (1) also comprises adjusting the pH of the system to 4-6 using acid after the reaction is completed, adding poor solvent of compound SEM130 to precipitate the solid, filtering or centrifuging to obtain residual solid, then beating with solvent, filtering, drying to obtain crude compound SEM130, which is directly used in step (2). The preferred poor solvent is one or more of acetonitrile, EA, MTBE, and MIBK. The solvent used for beating has poor solubility for SEM130 and good solubility for impurities, preferably one or more of MTBE, EA, acetonitrile, and MIBK.

In step (2), the solvent used for deprotection is a good solvent for the raw material (compound SEM130), and the amount of solvent used is such that the raw material can be completely dissolved in it. The preferred solvent is one or more combinations of dichloromethane, methyl *tert*-butyl ether, trifluoroacetic acid, TIS, acetonitrile, and water. The preferred reaction temperature for this step is 0-20 °C, and more preferably 0-10 °C. The preferred acid is selected from one or more of TFA, acetic acid, hydrochloric acid, hydrobromic acid, phosphoric acid, and methylsulfonic acid, and more preferably acetic acid, TFA. The volume to weight ratio of acid to compound SEM130 is 1-10 mL/g, and more preferably 3-7 mL/g. Step (2) also comprises after the reaction is completed, the reaction solution was poured into a poor solvent of Semaglutide at 0-20 °C, preferably 0-10 °C, to precipitate the solid, and centrifuging or filtering was then conducted. The preferred poor solvent is one or more of MTBE, acetonitrile, EA and MIBK, and more preferably MTBE. The crude solid product of Semaglutide was obtained by beating the obtained solid with solvent and filtration. The solvent used for beating has poor solubility for Semaglutide and good solubility for impurities, and is preferably one or more of MTBE, EA, acetonitrile, and MIBK, and more preferably MTBE. The solid crude product of Semaglutide was further purified by column chromatography.

In a preferred embodiment of the present invention, compound SEM120 can be prepared by the following step (1-d):
reacting the compound SEM115 and compound 2 in a solvent in the presence of a dehydrating agent to form compound SEM120, and the reaction formula is as follows: 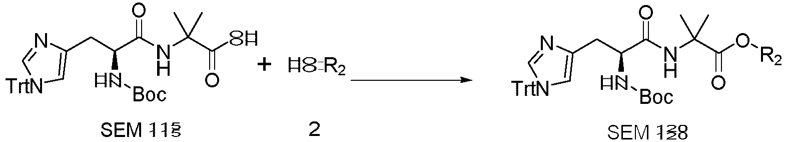
wherein, R₂ is *p*-nitrophenyl, methylsulfonyl, pentafluorophenyl or 5-norbornene-2,3-dicarboxamido.

In the process of preparing compound SEM120, the solvent used is a good solvent for all raw materials (compound SEM115 and compound 2), and the amount of solvent used is such that all raw materials can be completely dissolved in it. The preferred solvent is one or more combinations of N, N-dimethylformamide, N-methylpyrrolidone, N, N-dimethylacetamide, tetrahydrofuran, dichloromethane, acetone, acetonitrile, and water. The preferred dehydrating agent is one or more combinations of DCC, DIC, EDCI, and T₃P. The molar ratio of compound SEM115 to compound 2 is preferably is 1:1.0-1.4,and more preferably 1:1.0-1.2. The molar ratio of the dehydrating agent to compound SEM115 is 1.4-1.0:1, and more preferably 1.3-1.1:1. The preferred reaction temperature is 0-20 °C, and more preferably 0-10 °C. The process of preparing compound SEM120 also comprises that after the reaction is completed, the reaction solution is filtered and the filtrate is concentrated to dryness at a temperature not higher than 40 °C, such as 15-30 °C, to obtain compound 120. The compound 120 does not require further purification and can be directly used for the next reaction step.

In a preferred embodiment of the present invention, compound SEM110 can be prepared by the following step (1-c):
reacting the compound SEM100 and compound SEM105 in a solvent in the presence of an alkali to form SEM110, and the reaction formula is as follows: 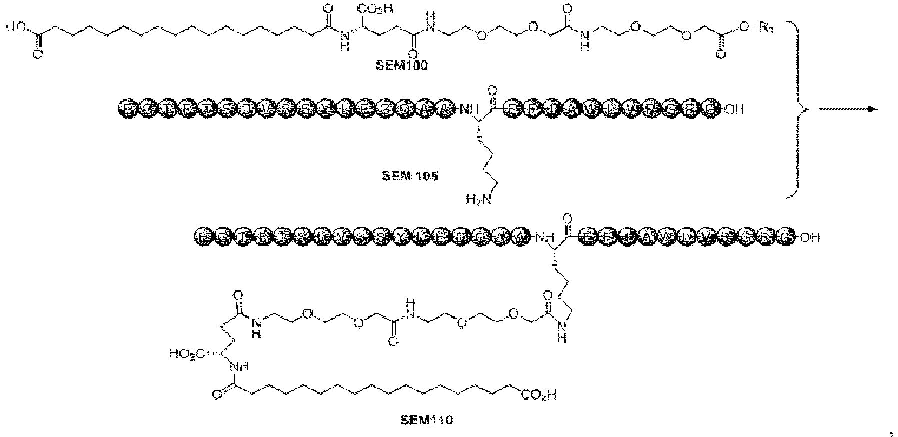
wherein, R₁ is *p*-nitrophenyl, methylsulfonyl, pentafluorophenyl or 5-norbornene-2,3-dicarboxamido.

In the preparation process of compound SEM110, the solvent used is a good solvent for all raw materials (compound SEM100 and compound SEM105), and the amount of solvent used is such that all raw materials can be completely dissolved in it. The preferred solvent is one or more combinations of N, N-dimethylformamide, N-methylpyrrolidone, N, N-dimethylacetamide, tetrahydrofuran, dichloromethane, acetone, acetonitrile, and water. The preferred alkali used is one or more combinations of carbonates, phosphates, bicarbonates, hydrophosphates, triethylamine, DBU, DMAP, and diisopropylethylamine, and more preferably diisopropylethylamine. The molar ratio of compound SEM100 to compound SEM105 is preferably 1.1-1.6:1, and more preferably 1.2-1.5:1. The preferred reaction temperature is 0-30 °C, and more preferably 0-15 °C. The molar ratio of compound SEM100 to compound SEM105 is preferably 1.3~1.5. The molar ratio of alkali to compound SEM105 is preferably 5-25:1, or the amount of alkali used is preferably to maintain the pH of the system at 10-11. The preferred reaction temperature is 0-30 °C, and more preferably 0-15 °C. The reaction solution containing compound SEM110 does not require further treatment and can be directly used for the next reaction step.

In a preferred embodiment of the present invention, compound SEM100 can be prepared by the following steps:
(1-a) reacting compound SEM80 and compound 1 in a solvent in the presence of a dehydrating agent to form SEM90, and the reaction formula is as follows: 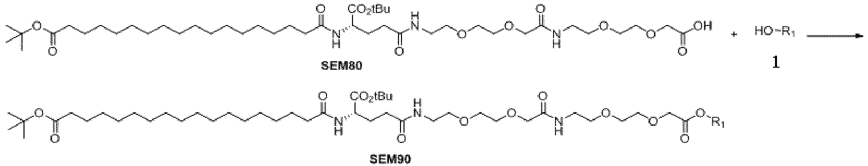 wherein, R₁ is *p*-nitrophenyl, methylsulfonyl, pentafluorophenyl or 5-norbornene-2,3-dicarboxamido;
(1-b) deprotecting compound SEM90 in a solvent in the presence of acid to form compound SEM100, and the reaction formula is as follows: 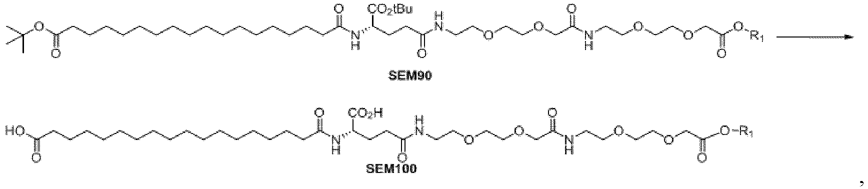 wherein, R₁ is *p*-nitrophenyl, methylsulfonyl, pentafluorophenyl or 5-norbornene-2,3-dicarboxamido.

In the preparation process of compound SEM100, in step (1-a), the solvent used is a good solvent for all raw materials (compound SEM80 and compound 1), and the amount of solvent used is such that all raw materials are completely dissolved in it. The preferred solvent is one or more combinations of N, N-dimethylformamide, N-methylpyrrolidone, N, N-dimethylacetamide, tetrahydrofuran, dichloromethane, acetone, acetonitrile, and water. The preferred dehydrating agent is one or more combinations of DCC, DIC, EDCI, and T₃P. The molar ratio of compound SEM80 to compound 1 is 1:1.0-1.4, and more preferably 1:1-1.2. The molar ratio of compound SEM80 to the dehydrating agent is 1:1.0-1.4, and more preferably 1:1.1-1.2. The preferred reaction temperature in this step is 0-20 °C, and more preferably 0-10 °C. This step also comprises that after the reaction is completed, the reaction solution is filtered, and the filtrate is concentrated at a temperature no higher than 40 °C, such as 15-30 °C, to obtain a concentrated solution containing the target compound, which is directly used for the next reaction step.

In the preparation process of compound SEM100, in step (1-b), the solvent used is a good solvent for the raw material (compound SEM90), and the amount of solvent used is such that the raw material can be completely dissolved in it. The preferred solvent is one or more combinations of dichloromethane, trifluoroacetic acid, TIS, acetonitrile, and water. The acid is selected from one or more of trifluoroacetic acid, acetic acid, hydrochloric acid, hydrobromic acid, phosphoric acid, and methylsulfonic acid. The preferred volume to weight ratio of acid to compound 90 is 1-10 mL/g. The preferred temperature for deprotection is 0-40 °C, and more preferably 10-20 °C. The preparation process of compound SEM100 also comprises that after deprotection is completed, a poor solvent of compound SEM100 is added dropwise to the reaction solution and stirred for 1-3 hours to allow the product to fully precipitate, and then subjected to filtration or centrifugation to obtain compound SEM100. The compound SEM100 can be directly used for the next reaction step without further purification. In this step, the preferred poor solvent is MTBE.

In the preparation method of Semaglutide of the present invention, the reaction progress of each step can be monitored using conventional monitoring methods in the field (such as TLC, HPLC, or NMR), and the reaction time is determined by the disappearance of the main raw material in the reaction solution. For example, in step (1-a), when compound SEM80 in the reaction solution was monitored and found disappeared, the reaction was considered completed. In step (1-d), when it was monitored and found that compound SEM115 in the reaction solution has disappeared, the reaction was considered completed.

In the description of the present invention, "more" means two or more.

In the description of the present invention, hydrochloric acid is an aqueous solution of hydrogen chloride gas, with a mass concentration of commonly used concentration, such as 5%~37.5%. The present invention preferably uses hydrochloric acid with a saturated hydrogen chloride concentration at ambient temperature. Hydrobromic acid is an aqueous solution of hydrogen bromide, with a mass concentration of commonly used concentration, such as 5%~68%. The present invention preferably uses hydrobromic acid with a saturated hydrobromic acid concentration at ambient temperature.

Compared with the prior art, the advantage and effect of the present invention lie in:
The preparation method of the present invention is easy to operate, and has mild reaction conditions. The side chain and short peptide are modified with nitrophenol ester, methanesulfonate, pentafluorophenol ester or HONB ester, avoiding impurities IMP1 and IMP2 caused by the use of HOSU. The final product Semaglutide is easy to purify, has high purity, and is suitable for industrial production.

In the present invention, the substances represented by abbreviations are shown in Table 1:

**Table 1**

| Abbreviation | Chinese Name | Abbreviation | Chinese Name |
|---|---|---|---|
| DCM | Dichloromethane | DMF | N, N-dimethylformamide |
| HONB | N-hydroxy-5-norbornen e-2,3-dicarboxamide | TIS | Triisopropylsilane |
| MTBE | Methyl *tert-*butyl ether | HOSU | N-hydroxysuccinimide |
| DCC | N,N'-dicyclohexylcarbo nimide | DIPEA | N,N-diisopropylethylamine |
| TFA | Trifluoroacetic acid | THF | Tetrahydrofuran |
| DIC | N,N'-diisopropylcarbodi imide | EA | Ethyl acetate |
| EDCI | 1-(3-dimethylaminoprop yl)-3-ethylcarbodiimide hydrochloride | MIBK | 4-Methyl-2-pentanone |
| T₃P | 1-Propylphosphonic anhydride | Ms₂O | Methylsulfonic anhydride |

The present invention will be further explained in detail below in combination with examples. The examples of the present invention are only used to illustrate the technical solution of the present invention, and the essence and scope of the present invention are not limited to them. Unless otherwise specified, percentages and parts are percentages and parts by weight.

The reagents used in the following examples, unless otherwise specified, are commercially available.

SEM80 and SEM115 are both purchased from Chengdu Pukang Biotechnology Co., Ltd.

The preparation method of SEM105 refers to Chinese patent application number CN200880124022.1.

The manufacturer models of the high-performance liquid chromatography used in the following examples are Agilent 1260II and Waters e2695+2698; The manufacturer model of the mass spectrometer used is Waters H-Class.

### Example 1: Preparation of compound SEM100A using SEM80 and HONB.

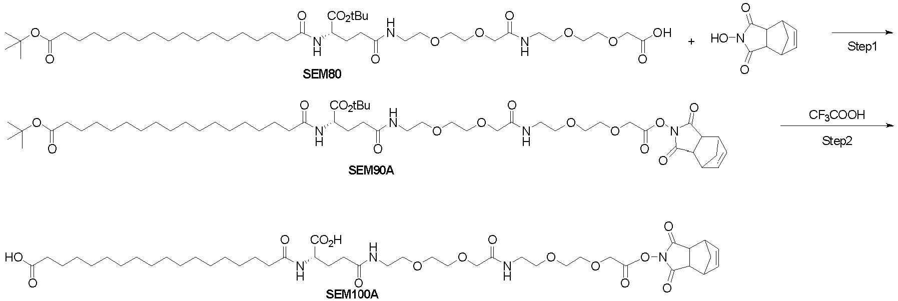

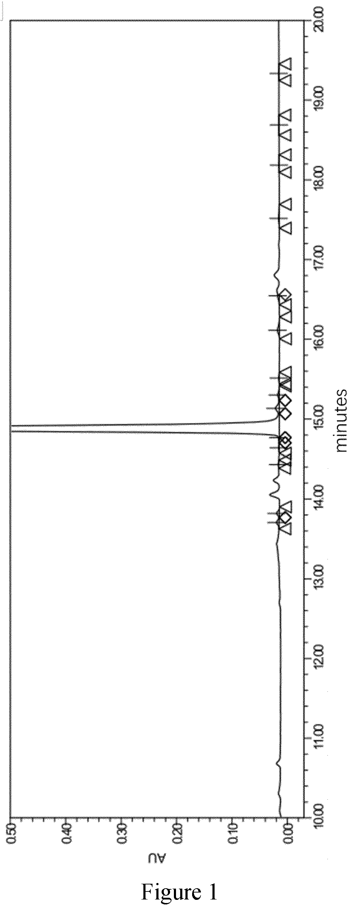

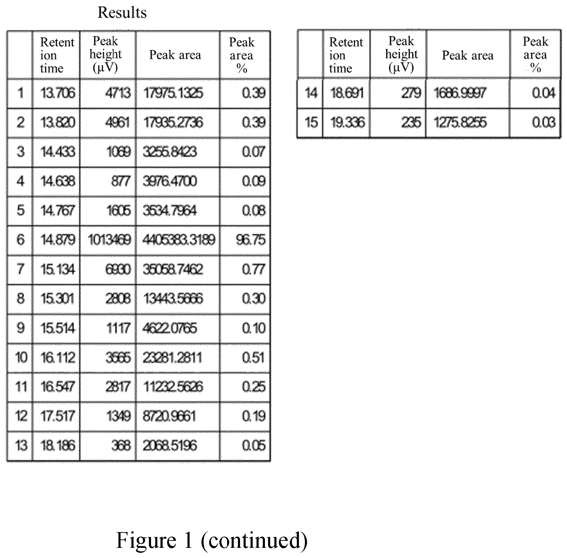

(1) Under nitrogen protection, the temperature was controlled at 15-30 °C. Compound SEM80 (10 g, 11.82 mmol, 1.0 eq.), DCM (80 mL, 8.0 vol.), and HONB (2.2 g, 12.41 mmol, 1.05 eq.) were added in sequence and the mixture was stirred to dissolve. The resulting mixture was cooled to 0-10 °C under an ice water bath, and added with DCC (2.9 g, 14.18 mmol, 1.2 eq.). After addition, the mixture was kept at 0-10 °C until the reaction was completed. The system was filtered and the filter cake was discarded. The filtrate was concentrated at 15-30 °C to 1.5-2.0 vol. (i.e. 15-20 mL) to obtain a DCM solution of compound SEM90A.
(2) The DCM solution of SEM90A was cooled to 0-10 °C. The temperature was controlled at 0-10 °C, and DCM (10 mL, 1.0 vol.) and TFA (40 mL, 4.0 vol.) were added in sequence. After addition, the system was warmed to 10-20 °C and reacted until the conversion of SEM90 was complete. MTBE (200 mL, 20 vol.) was added dropwise at 10-20 °C. After stirring for 2 hours, the resulting mixture was filtered, and the filter cake was washed with MTBE (50 mL, 5 vol.) and vacuum-dried to obtain 11.2 g of compound SEM100A as a white solid. The product SEM100A was an active ester, and the sample processing method was as follows: SEM100A was taken, quenched with excess cyclohexylamine, and subjected to HPLC and mass spectrometry detection. The HPLC spectrum was shown in Figure 1. Liquid phase purity: 96.75%, molar yield: 86.60%.

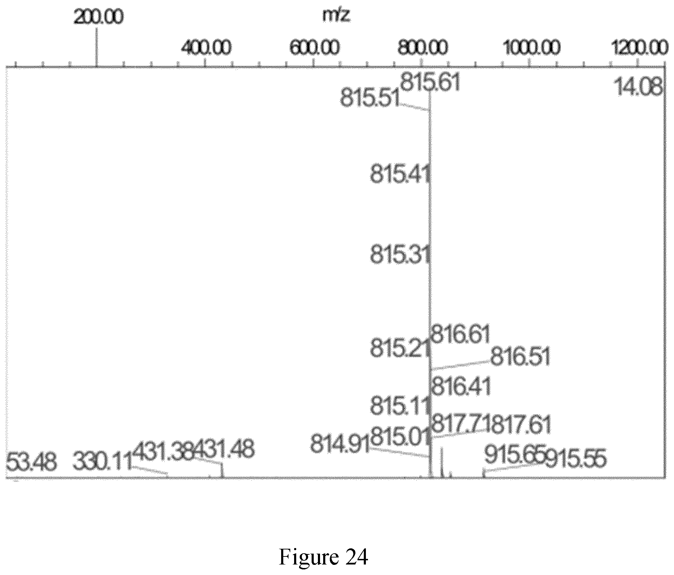

Mass spectrometry information: m/z=815.61 (M+H)⁺. A typical mass spectrum was shown in Figure 24.

### Example 2: Preparation of compound SEM100B using SEM80 and pentafluorophenol

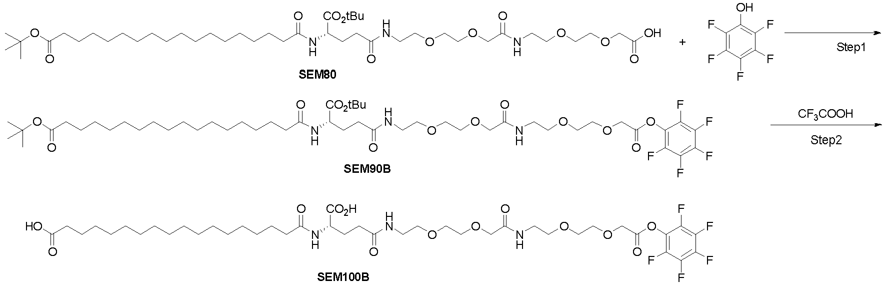

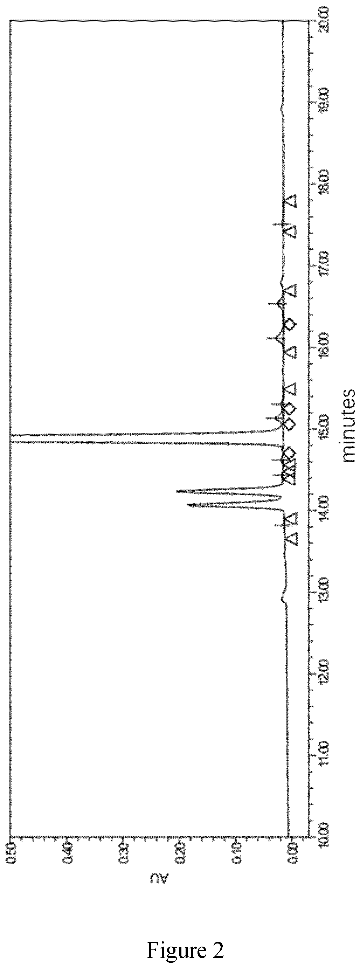

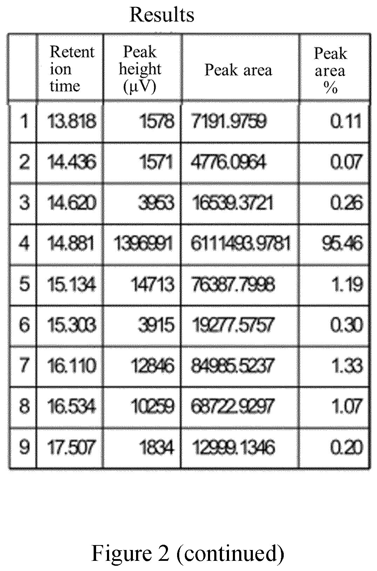

(1) Under nitrogen protection, the temperature was controlled at 15-30 °C. SEM80 (10 g, 11.82 mmol, 1.0 eq.), DCM (80 mL, 8.0 vol.), and pentafluorophenol (2.28 g, 12.41 mmol, 1.05 eq.) were added in sequence and the mixture was stirred to dissolve. The resulting mixture was cooled to 0-10 °C in an ice water bath, DCC (2.9 g, 14.18 mmol, 1.2 eq.) was added, and after addition, the mixture was kept at 0-10 °C until the reaction was completed. The system was filtered and the filter cake was discarded. The filtrate was concentrated at 15-30 °C to 1.5-2.0 vol. (i.e. 15-20 mL) to obtain a DCM solution of compound SEM90B.
(2) The DCM solution of compound SEM90B was cooled to 0-10 °C. The temperature was controlled at 0-10 °C, and DCM (10 mL, 1.0 vol.) and TFA (40 mL, 4.0 vol.) were added in sequence. After addition, the system was warmed to 10-20 °C and reacted until the conversion of SEM90B was complete. MTBE (200 mL, 20 vol.) was added dropwise at 10-20 °C. After stirring for 2 hours, the resulting mixture was filtered, and the filter cake was washed with MTBE (50 mL, 5 vol.) and vacuum-dried to obtain 12.46 g of SEM100B as an off-white solid. The product was an active ester, and the sample processing method was as follows: SEM100B was taken, quenched with excess cyclohexylamine, and subjected to HPLC and mass spectrometry detection. The HPLC spectrum was shown in Figure 2. Liquid phase purity: 95.46%, molar yield: 88.96%.

Mass spectrometry information: m/z=815.61 (M+H)⁺. A typical mass spectrum was shown in Figure 24.

### Example 3: Preparation of compound SEM120A using SEM115 and pentafluorophenol

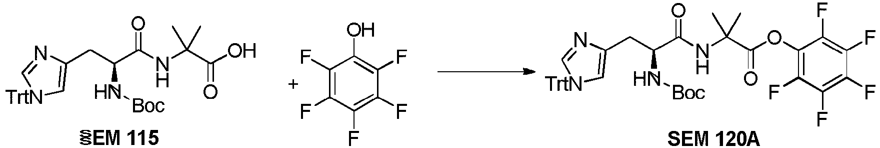

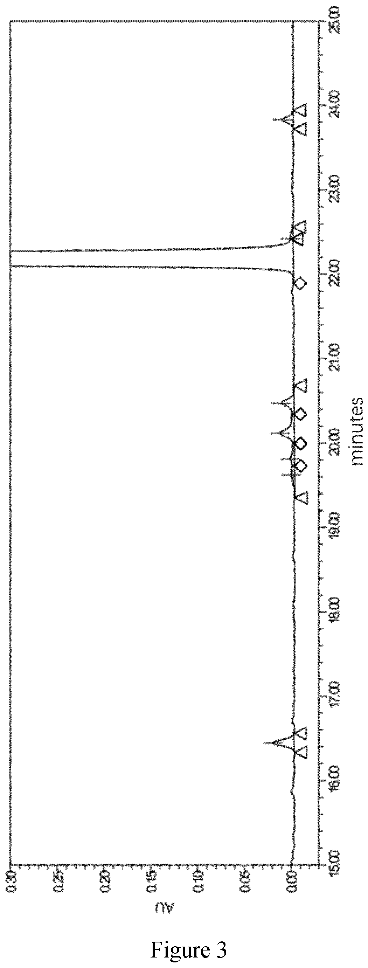

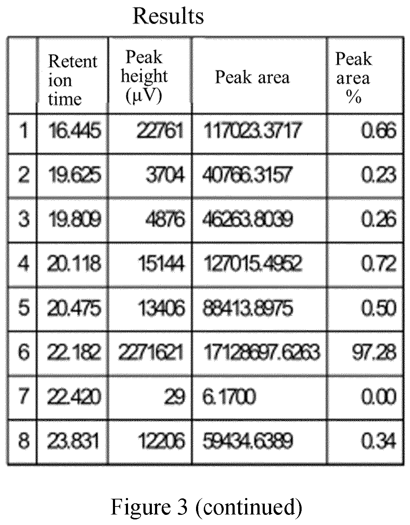

Under nitrogen protection, the temperature was controlled at 15-30 °C. SEM115 (10 g, 17.22 mmol, 1.0 eq.), THF (80 mL, 8.0 vol.), and pentafluorophenol (3.33g, 18.08 mmol, 1.05 eq.) were added in sequence and the mixture was stirred to dissolve. The resulting mixture was cooled to 0-10 °C in an ice water bath, DCC (4.26 g, 20.66 mmol, 1.2 eq.) was added, and after addition, the mixture was kept at 0-10 °C until the reaction was completed. The system was filtered and the filter cake was discarded. The filtrate was concentrated at 15-30 °C to dryness to obtain 13.38 g of SEM120A as an off-white solid. The product was an active ester, and the sample processing method was as follows: SEM120A was taken, quenched with excess cyclohexylamine, and subjected to HPLC and mass spectrometry detection. The HPLC chromatogram was shown in Figure 3. Liquid phase purity: 97.28%, molar yield: 90.65%.

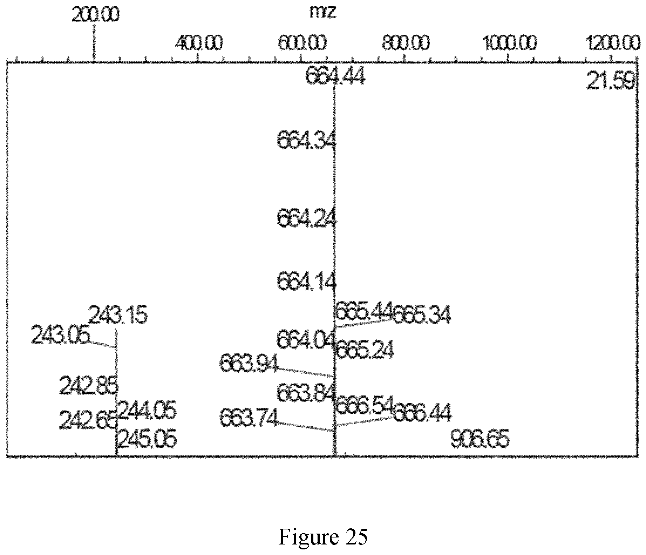

Mass spectrometry information: m/z=664.44 (M+H)⁺. A typical mass spectrum was shown in Figure 25.

### Example 4: Preparation of compound SEM120B using compound SEM115 and HONB

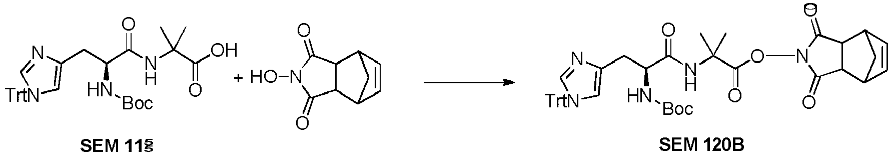

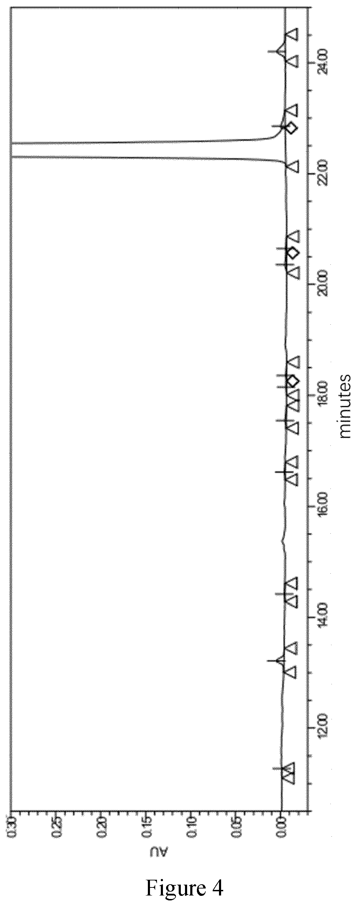

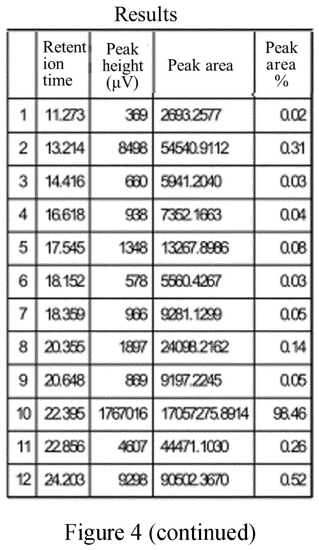

Under nitrogen protection, the temperature was controlled at 15-30 °C. SEM115 (10 g, 17.16 mmol, 1.0 eq.), THF (80 mL, 8.0 vol.), and HONB (3.22g, 18.02 mmol, 1.05 eq.) were added in sequence and the mixture was stirred to dissolve. The resulting mixture was cooled to 0-10 °C in an ice water bath, DCC (4.25 g, 20.59 mmol, 1.2 eq.) was added, and after addition, the mixture was kept at 0-10 °C until the reaction was completed. The system was filtered and the filter cake was discarded. The filtrate was concentrated at 15-30 °C to dryness to obtain 14.07 g of SEM120B as an off-white solid. The product was an active ester, and the sample processing method was as follows: SEM120B was taken, quenched with excess cyclohexylamine, and subjected to HPLC and mass spectrometry detection. The HPLC chromatogram was shown in Figure 4. Liquid phase purity: 98.46%, molar yield: 94.20%.

Mass spectrometry information: m/z=664.44 (M+H)⁺. A typical mass spectrum was shown in Figure 25.

### Example 5: Preparation of compound SEM120C using compound SEM115 and methylsulfonic anhydride

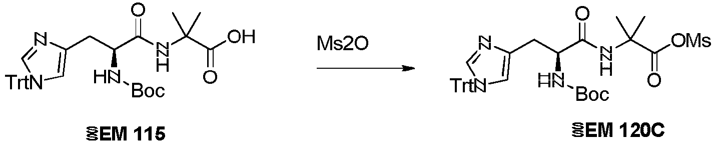

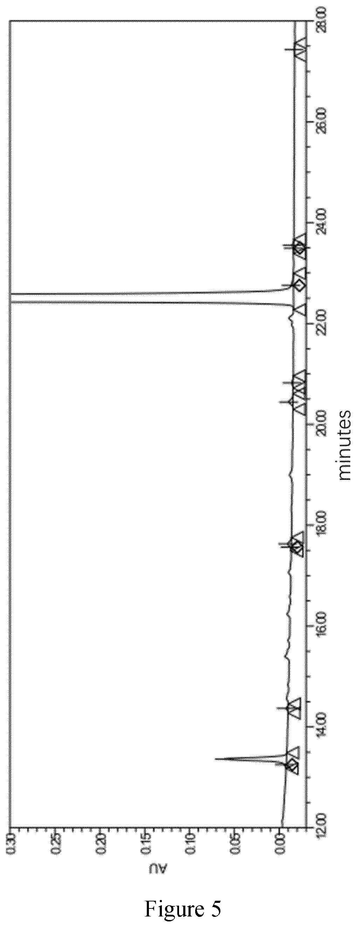

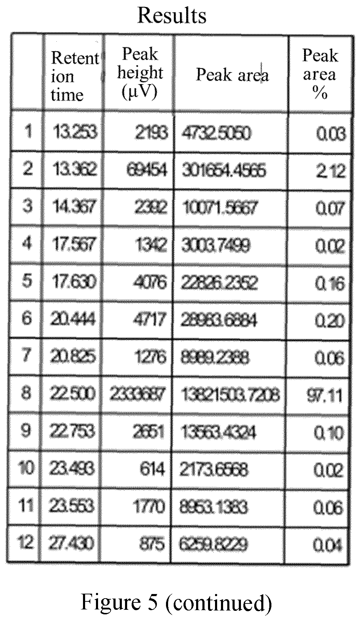

Under nitrogen protection, the temperature was controlled at 15-30 °C. SEM115 (10 g, 17.16 mmol, 1.0 eq.), THF (80 mL, 8.0 vol.), and DIPEA (5.54g, 42.90 mmol, 2.50 eq.) were added in sequence and the mixture was stirred to dissolve. The resulting mixture was cooled to 0-10 °C in an ice water bath, a solution of methylsulfonic anhydride (3.59 g, 20.59 mmol, 1.2 eq.) in THF (20 mL, 2.0 vol.) was added dropwise, and after addition, the mixture was kept at 0-10 °C until the reaction was completed. The system was concentrated at 15-30 °C to dryness to obtain 12.38 g of SEM120C as an off-white solid. The product was an active ester, and the sample processing method was as follows: SEM120C was taken, quenched with excess cyclohexylamine, and subjected to HPLC detection. The HPLC chromatogram was shown in Figure 5. Liquid phase purity: 97.11%, molar yield: 94.54%.

Mass spectrometry information: m/z=664.44 (M+H)⁺. A typical mass spectrum was shown in Figure 25.

### Example 6: Preparation of compound SEM130 using compound SEM100A, GLP-1 (9-37) peptide, and compound 120A

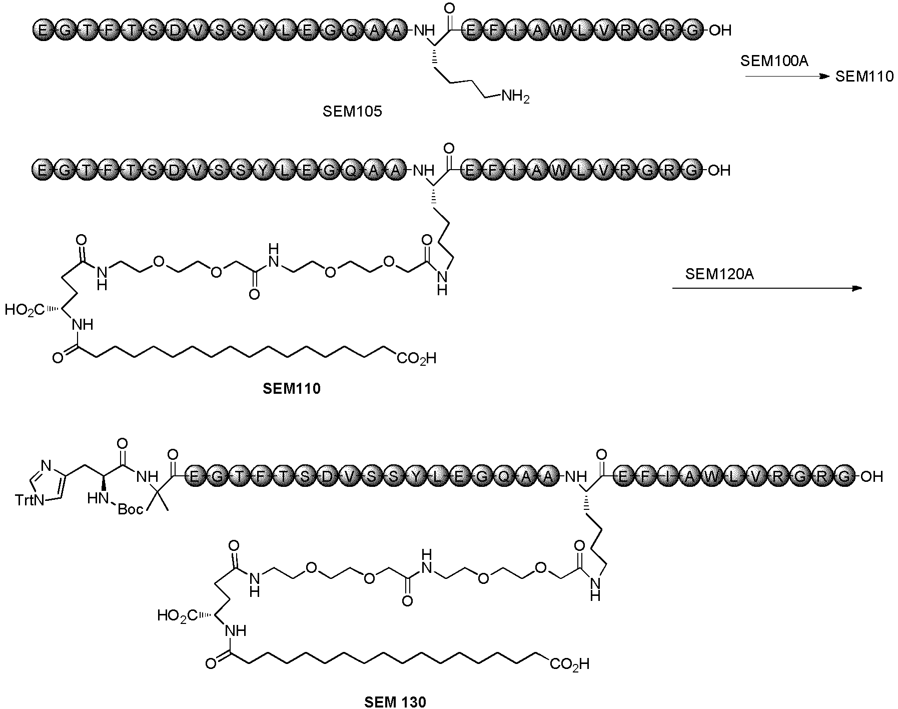

(1) Under nitrogen protection, the temperature was controlled at 15-30 °C. GLP-1 (9-37) peptide (i.e. compound SEM105) (1.0 g, 0.315 mmol, 1.0 eq.), THF (8 mL, 8.0 vol.), and purified water (20 mL, 20 vol.) were added in sequence. The resulting mixture was cooled to 0-15 °C in an ice water bath, DIPEA (0.81 g, 6.30 mmol, 20 eq.) was added, and the resulting mixture was stirred to dissolve. The temperature was controlled at 0-15 °C and a solution of SEM100A (0.48 g, 0.47 mmol, 1.5 eq.) in THF was added dropwise. After addition, the mixture was kept at 0-15 °C until the reaction was completed. The compound SEM110/THF/aqueous solution was obtained and directly used for the next reaction step.

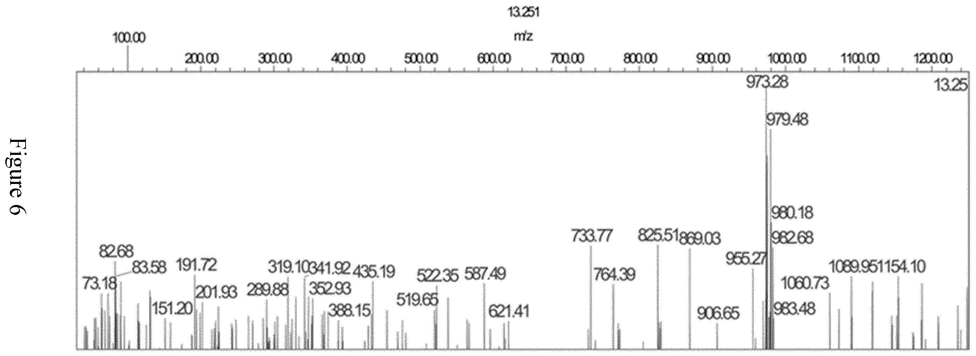

Mass spectrometry information: m/z=973.28 (M+4H)⁴⁺. A typical mass spectrum of compound SEM110 was shown in Figure 6.

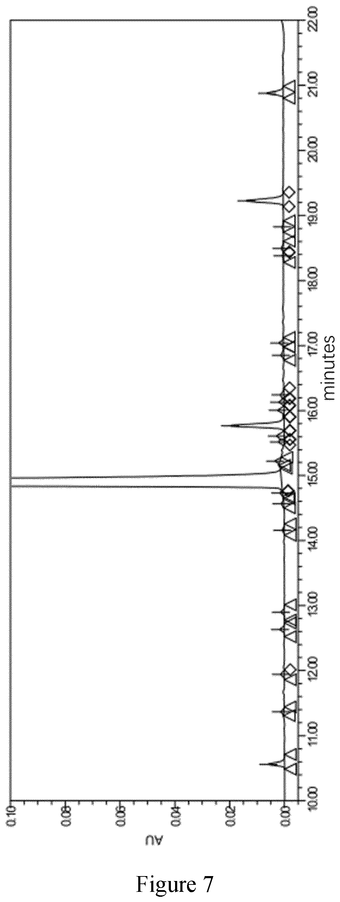

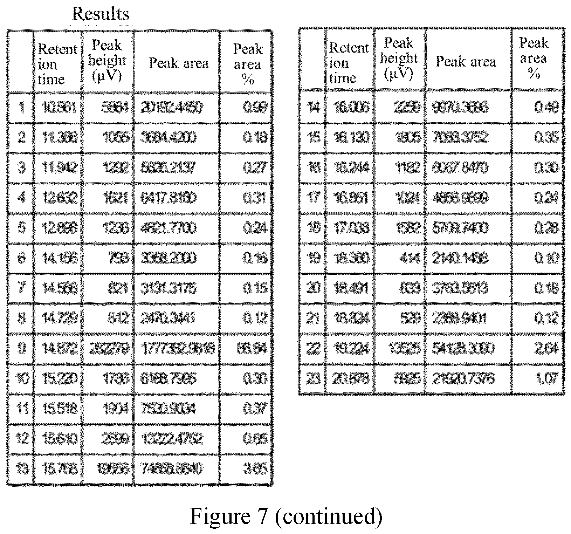

HPLC detection was performed, and the HPLC chromatogram was shown in Figure 7. Liquid phase purity: 86.84%.

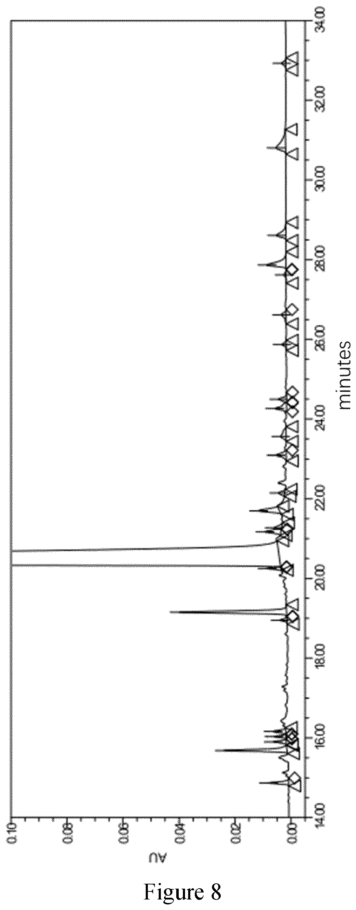

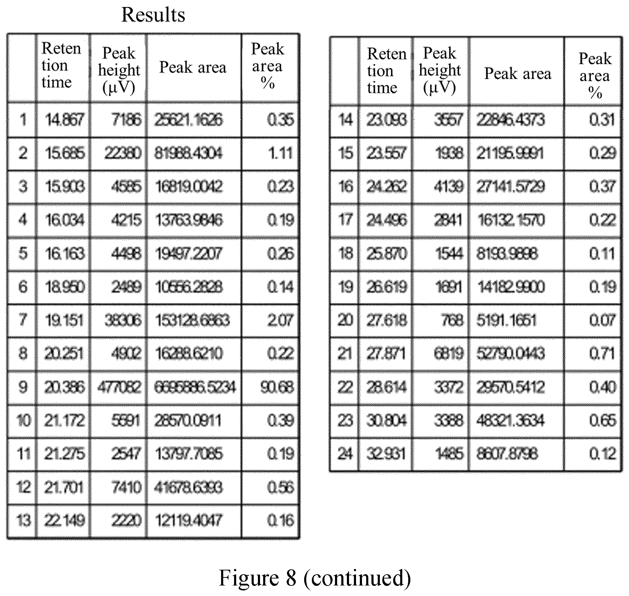

(2) Acetic acid (0.38g, 6.30mmol, 20 eq.) was added to the compound SEM110/THF/aqueous solution, and the resulting mixture was stirred at 0-15 °C for 1.0 hour. DIPEA was added to adjust the pH to 8-9, and the resulting mixture was cooled to 0-15 °C in an ice water bath. Compound SEM120A (0.71g, 0.945 mmol, 3 eq.)/THF solution was added, and the mixture was kept at 0-15 °C until the reaction was completed. Acetic acid was added dropwise to adjust the pH to 4-6. The temperature was warmed to 15-35 °C and the mixture was concentrated to a remaining volume of about 20-23 vol., with a large amount of solid precipitated. The resulting mixture was centrifuged and dried to obtain crude SEM130, which was then pulped with MTBE (150 mL, 15 vol.) at 15-25 °C for 2-3 hours, and filtered. The filter cake was dried in vacuum to obtain 1.36g of crude compound SEM130 as an off-white solid. The product was subjected to HPLC detection, and the HPLC chromatogram was shown in Figure 8. Liquid phase purity: 90.68%, molar yield: 83.52%.

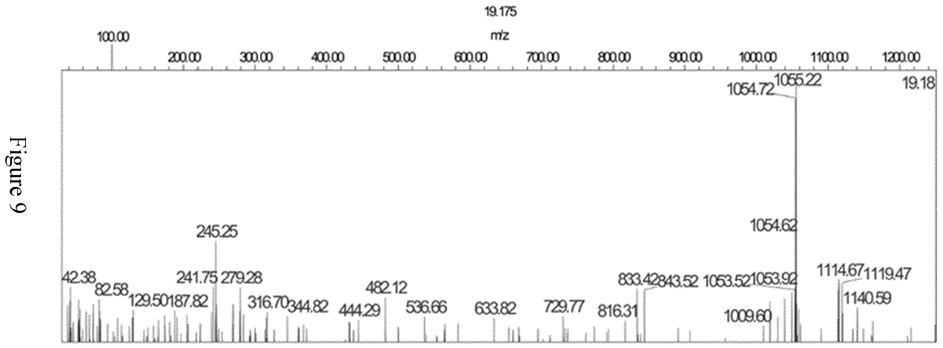

Mass spectrometry information: m/z=1114.27 (M+4H)⁴⁺. A typical mass spectrum of compound SEM130 was shown in Figure 9.

### Example 7: Preparation of compound SEM130 using compound SEM100A, GLP-1 (9-37) peptide, and compound 120B.

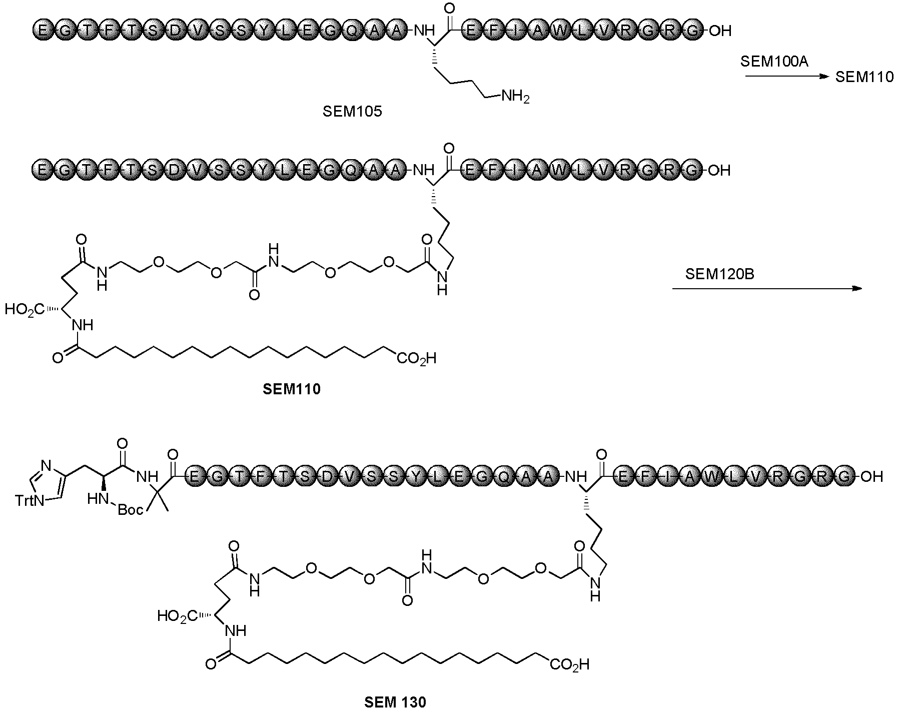

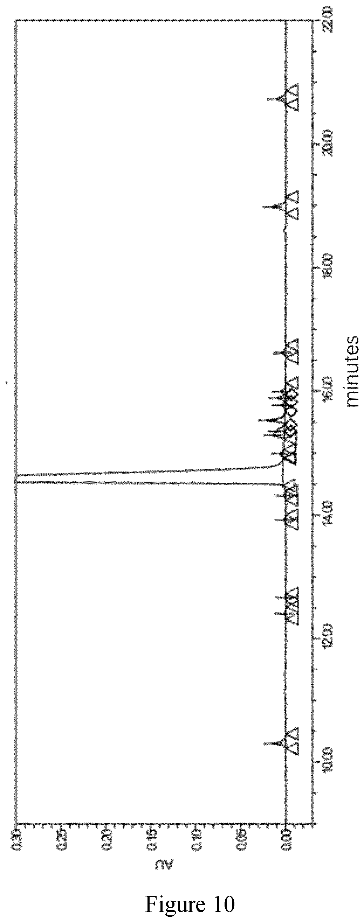

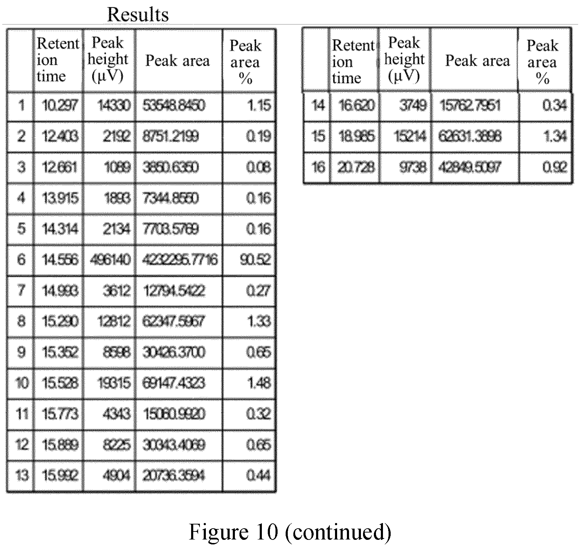

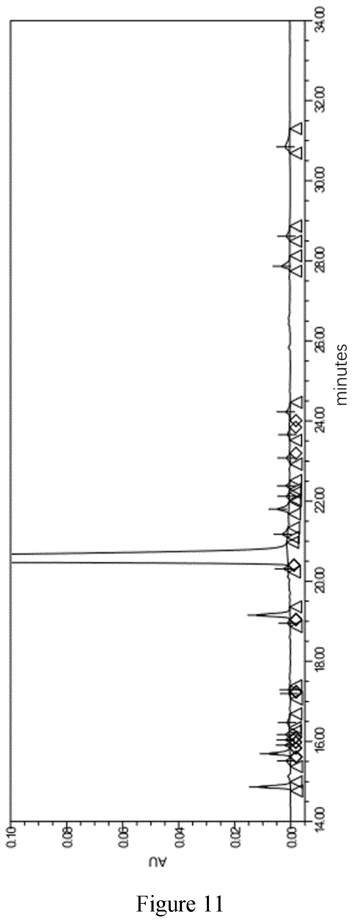

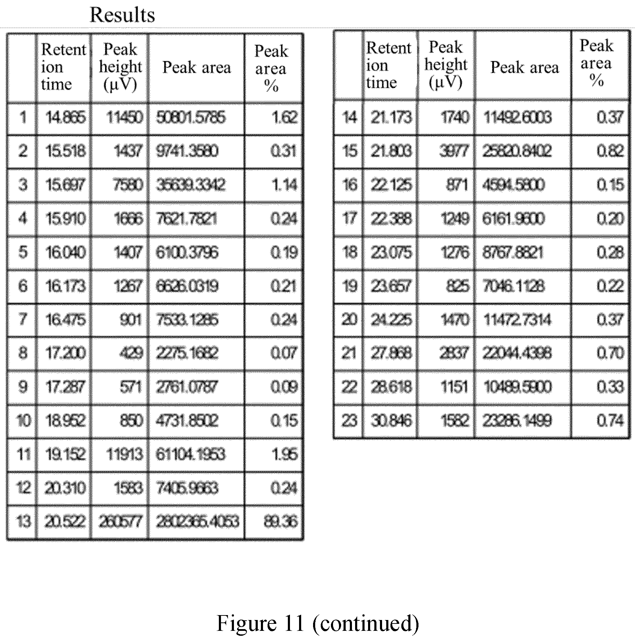

(1) Under nitrogen protection, the temperature was controlled at 15-30 °C. GLP-1 (9-37) peptide (1.0 g, 0.315 mmol, 1.0 eq.), THF (8 mL, 8.0 vol.), and purified water (20 mL, 20 vol.) were added in sequence. The resulting mixture was cooled to 0-15 °C in an ice water bath, DIPEA (0.81 g, 6.30 mmol, 20 eq.) was added, and the resulting mixture was stirred to dissolve. The temperature was controlled at 0-15 °C and a solution of SEM100A (0.48 g, 0.47 mmol, 1.5 eq.) in THF was added dropwise. The temperature was maintained until the reaction was completed, and acetic acid was added dropwise to adjust the pH to 4-6. The mixture was concentrated at 15-35 °C to a remaining volume of about 20-23 vol., with a large amount of solid precipitated. The resulting mixture was centrifuged and dried to obtain a crude compound SEM110 containing water, which was pulped with MTBE (150 mL, 15 vol.) at 15-25 °C for 2-3 hours, and repeatedly pulped with MTBE (150 mL, 15 vol.) at 15-25 °C for 2-3 hours and then filtered, and the filter cake was dried in vacuum to obtain an anhydrous crude compound SEM110 as 1.31g of an off-white solid. The product was subjected to HPLC detection, and the HPLC chromatogram was shown in Figure 10. Liquid phase purity: 90.52%. The product was added according to the theoretical amount for the next reaction step.
(2) DMF (15 mL, 15 vol.) was added to the anhydrous crude compound SEM110 (1.0g, 0.256 mmol, 1 eq.), DIPEA was added to adjust the pH to 8-9, and the resulting mixture was stirred to dissolve. Compound SEM120B (0.48g, 0.64mmol, 2.5 eq.) was added and the mixture was kept at 0-15 °C until the reaction was completed. Acetic acid was added dropwise to adjust the pH to 4-6. Acetonitrile (45 mL, 35 vol.) was added dropwise, and a large amount of solid precipitated. The resulting mixture was centrifuged to obtain crude SEM130, which was pulped with MTBE (150 mL, 15 vol.) at 15-25 °C for 2-3 hours, and filtered. The filter cake was dried in vacuum to obtain 1.19g of SEM130 as an off-white solid. The product was subjected to HPLC detection, and the HPLC chromatogram was shown in Figure 11. Liquid phase purity: 89.36%, molar yield: 90.36%.

Mass spectrometry information: m/z=1114.27 (M+4H) ⁴⁺.

### Example 8: Preparation of compound SEM130 using compound SEM100A, GLP-1 (9-37) peptide, and compound 120C

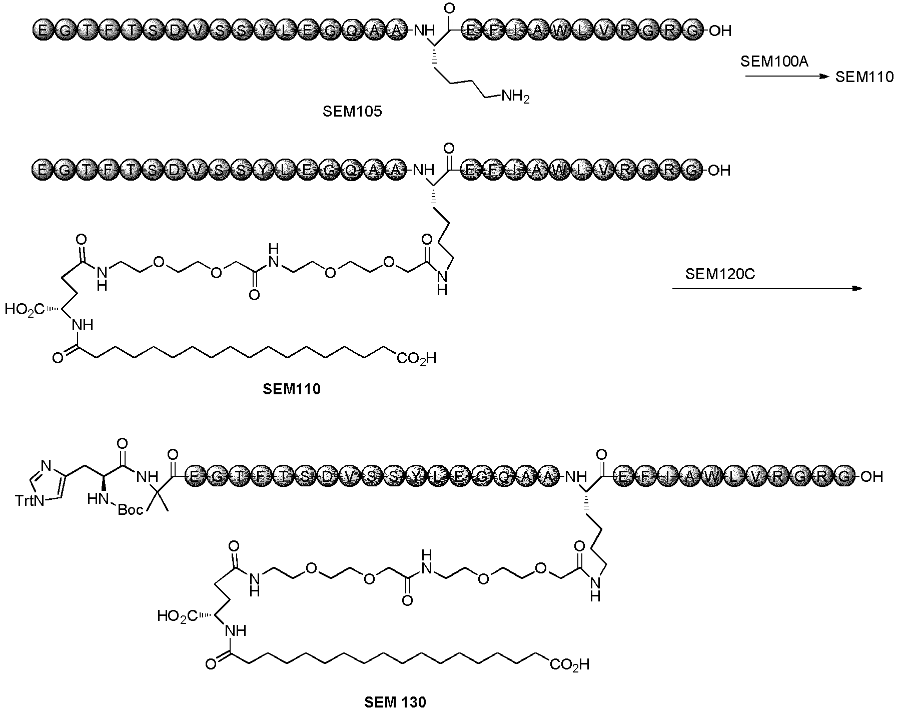

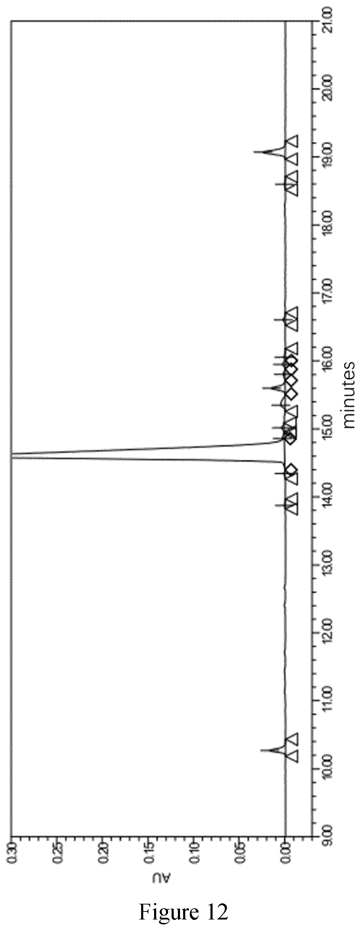

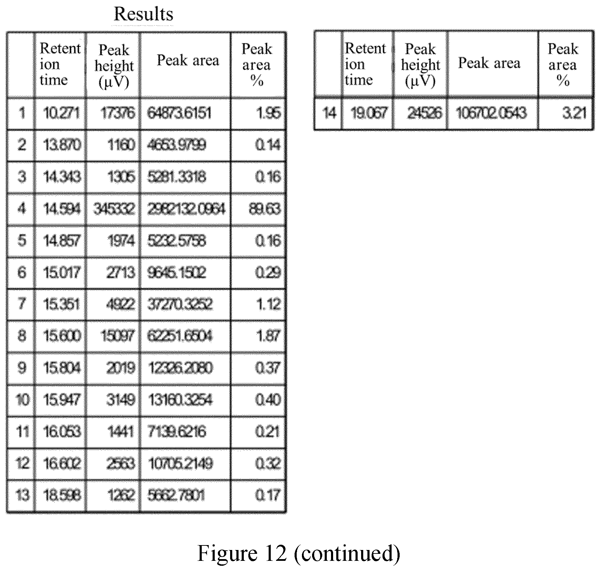

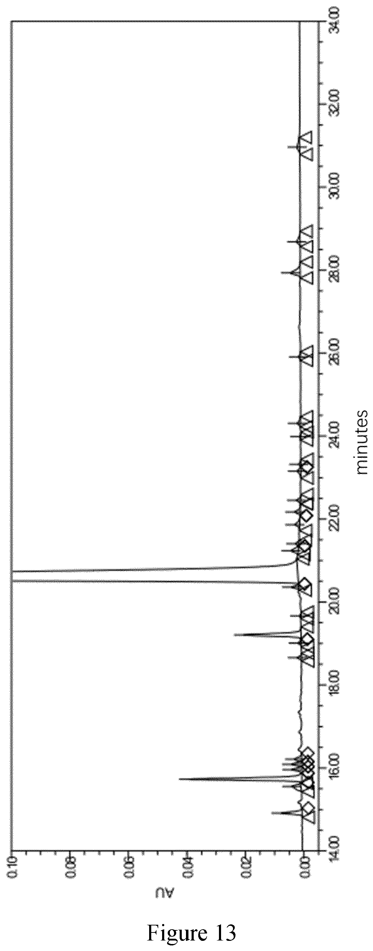

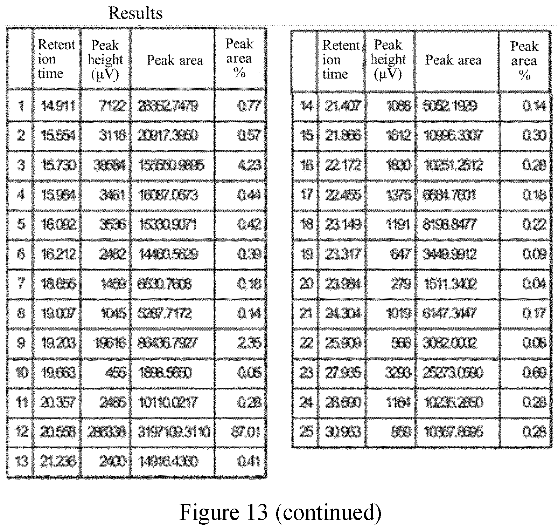

(1) The preparation process of SEM110 was similar to that in Example 7; the product was subjected to HPLC detection, and the HPLC chromatogram was shown in Figure 12. Liquid phase purity: 89.63%.
(2) DMF (150 mL, 15 vol.) was added to the anhydrous crude compound SEM110 (1.0g, 0.256 mmol, 1 eq.), DIPEA was added to adjust the pH to 8-9, and the resulting mixture was stirred to dissolve. Compound SEM120C (0.42g, 0.64mmol, 2.5 eq.) was added and the mixture was kept at 0-15 °C until the reaction was completed. Acetic acid was added dropwise to adjust the pH to 4-6. Acetonitrile (45 mL, 35 vol.) was added dropwise, and a large amount of solid precipitated. The resulting mixture was centrifuged to obtain crude compound SEM130, which was pulped with MTBE (150 mL, 15 vol.) at 15-25 °C for 2-3 hours and filtered. The filter cake was dried in vacuum to obtain 1.21g of SEM130 as an off-white solid. The product was subjected to HPLC detection, and the HPLC chromatogram was shown in Figure 13. Liquid phase purity: 87.01%, molar yield: 91.45%.

Mass spectrometry information: m/z=1114.27 (M+4H) ⁴⁺.

### Example 9: Preparation of compound SEM120D using compound SEM115 and p-nitrophenol

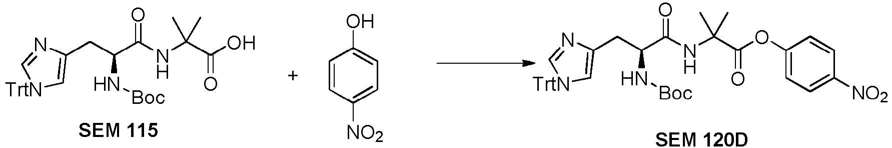

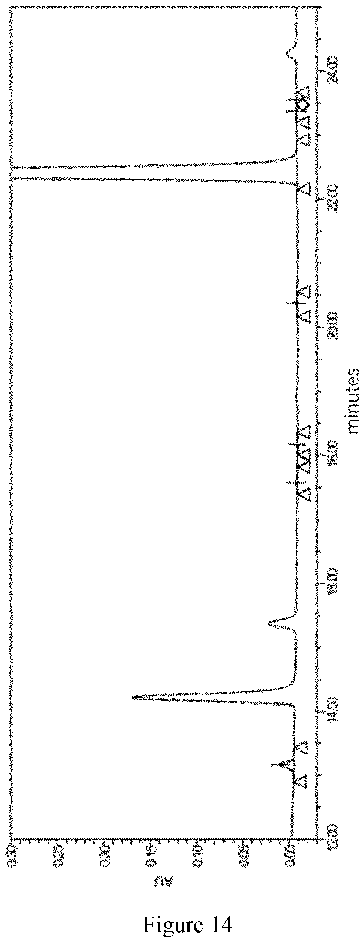

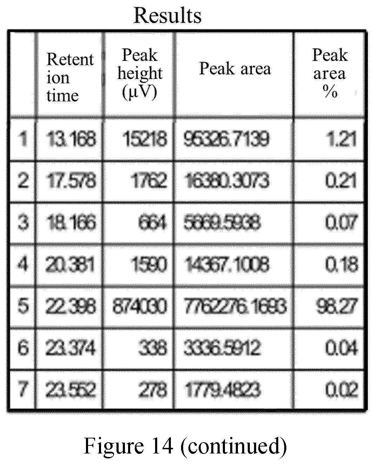

Under nitrogen protection, the temperature was controlled at 15-30 °C. SEM115 (10 g, 17.16 mmol, 1.0 eq.), THF (80 mL, 8.0 vol.), and *p*-nitrophenol (2.86g, 20.59 mmol, 1.2 eq.) were added in sequence and the mixture was stirred to dissolve. The resulting mixture was cooled to 0-10 °C in an ice water bath, a solution of DCC (4.25 g, 20.59 mmol, 1.2 eq.) in THF (20 mL, 2.0 vol.) was added dropwise, and after addition, the mixture was kept at 0-10 °C until the reaction was completed. The system was concentrated at 15-30 °C to dryness to obtain 12.56 g of SEM120D as a yellow solid. The product was subjected to HPLC detection. The HPLC chromatogram was shown in Figure 14. Liquid phase purity: 98.27%, molar yield: 93.89%.

Mass spectrometry information: m/z=664.44 (M+H)⁺. A typical mass spectrum was shown in Figure 25.

### Example 10: Preparation of compound SEM130 using compound SEM100A, GLP-1 (9-37) peptide, and compound SEM120D

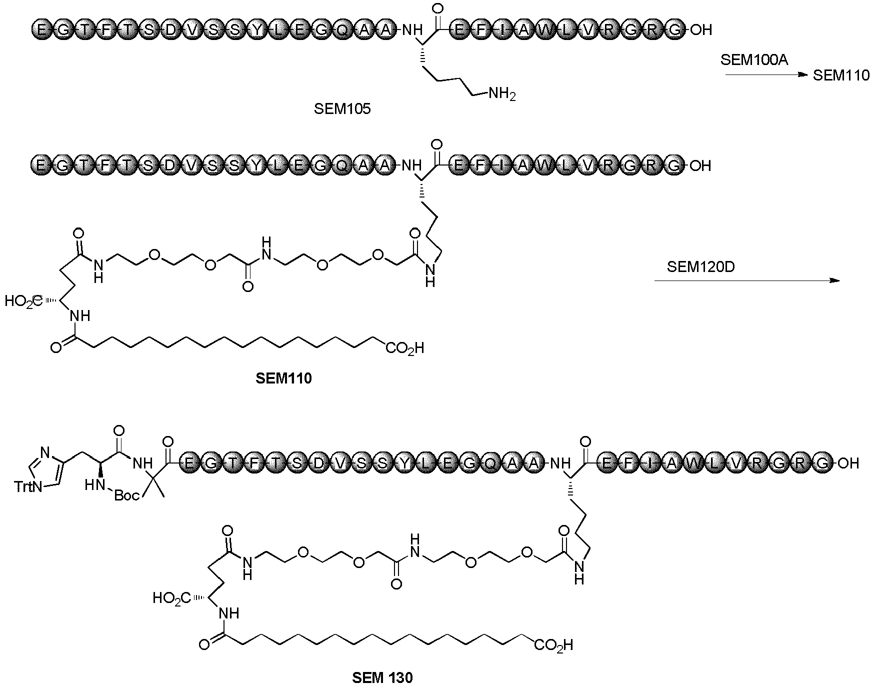

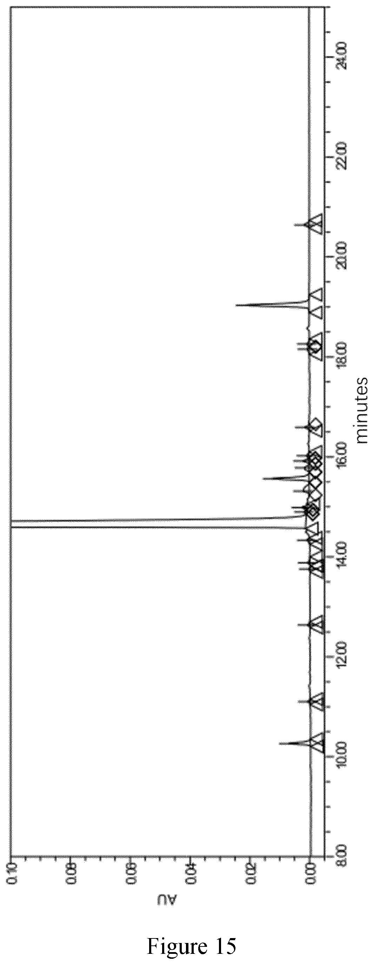

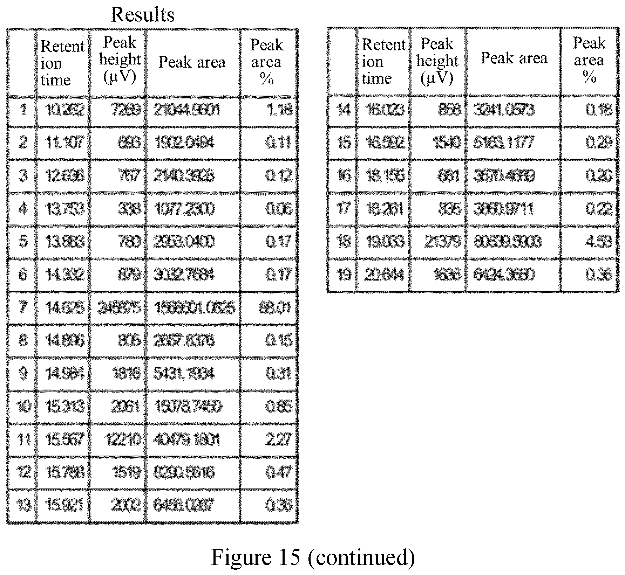

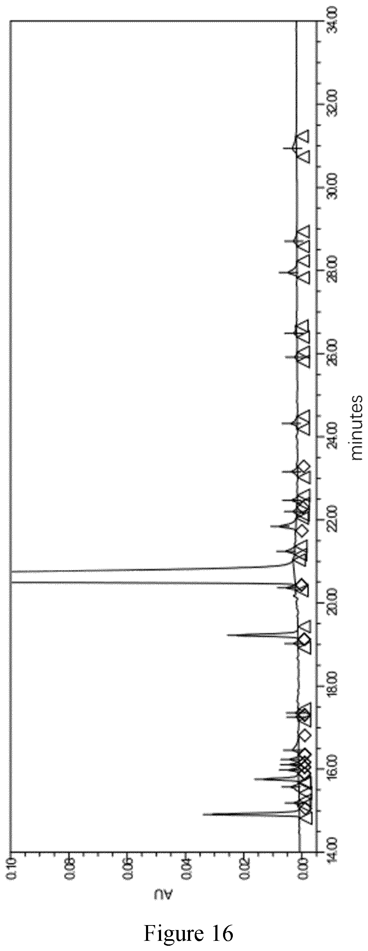

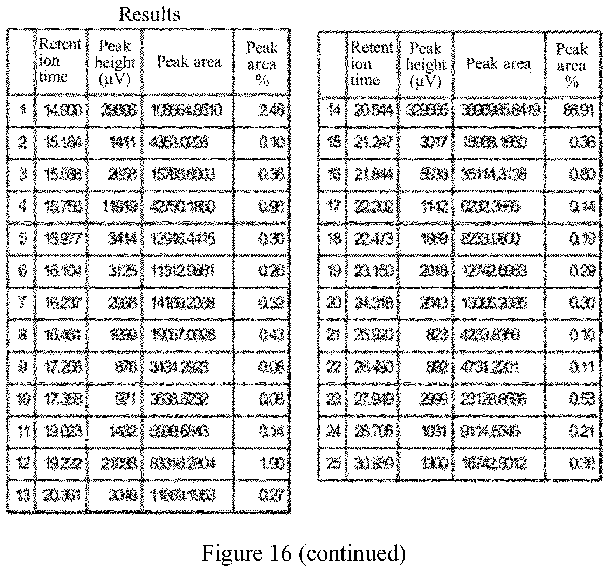

(1) The preparation process of compound SEM110 was similar to that in Example 7; the product was subjected to HPLC detection, and the HPLC chromatogram was shown in Figure 15. Liquid phase purity: 88.01%.
(2) The anhydrous crude SEM110 (1.0g, 0.257 mmol, 1 eq.) was added to DMF (150 mL, 15 vol.), DIPEA was added to adjust the pH to 8-9, and the resulting mixture was stirred to dissolve. SEM120D (0.72g, 1.03mmol, 4 eq.) was added and the mixture was kept at 0-15 °C until the reaction was completed. Acetic acid was added dropwise to adjust the pH to 4-6. Acetonitrile (45 mL, 35 vol.) was added dropwise, and a large amount of solid precipitated. The resulting mixture was centrifuged to obtain crude SEM130, which was pulped with MTBE (150 mL, 15 vol.) at 15-25 °C for 2-3 hours and filtered. The filter cake was dried in vacuum to obtain 1.24g of SEM130 as an off-white solid. The product was subjected to HPLC detection, and the HPLC chromatogram was shown in Figure 16. Liquid phase purity: 88.91%, molar yield: 95.38%.

Mass spectrometry information: m/z=1114.27 (M+4H) ⁴⁺.

### Example 11: Preparation of Semaglutide

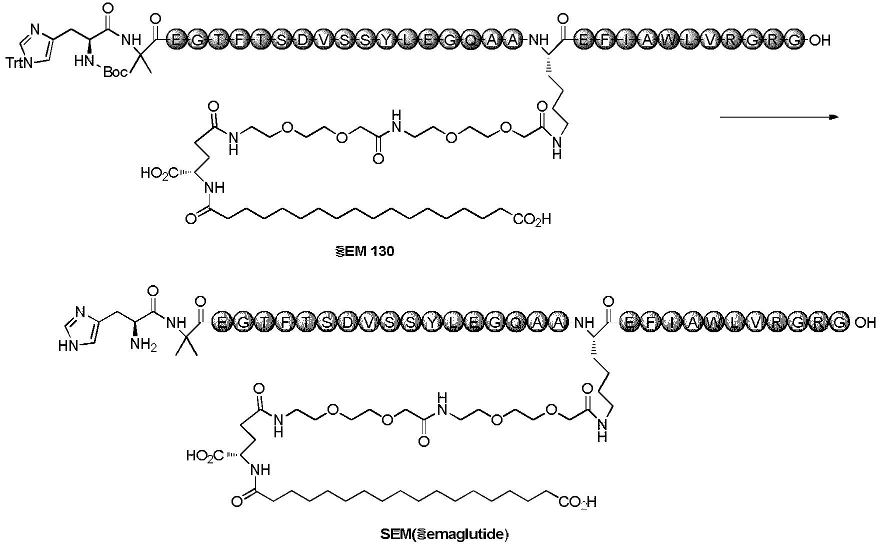

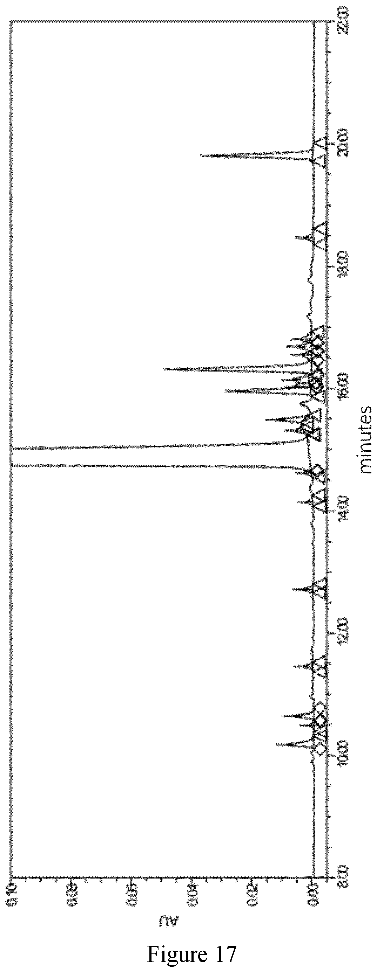

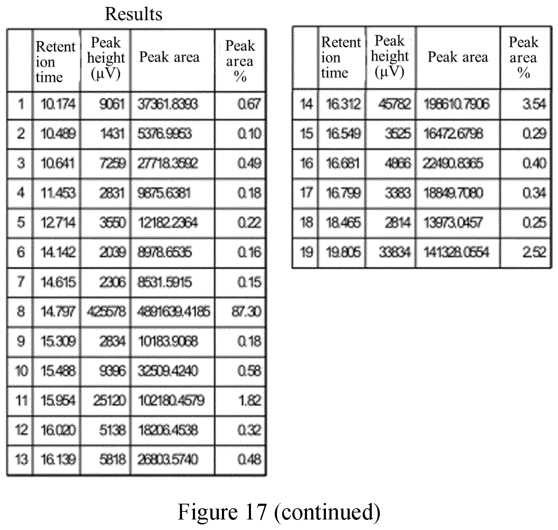

(1) Under nitrogen protection, the temperature was controlled at 0-10 °C. Compound SEM130 (1.0 g, 0.224 mmol, 1.0 eq.) prepared in Example 6, DCM (5 mL, 5.0 vol.), TIS (0.25 mL, 0.25 vol.), and TFA (5 mL, 5.0 vol.) were added in sequence and the mixture was stirred to dissolve. The system was kept at 0-10 °C until the reaction was completed. The system was controlled at the temperature of 0-10 °C and then added dropwise to MTBE (45 mL, 45 vol.) which was pre-cooled to 0-10 °C to precipitate an off-white solid. The resulting mixture was centrifuged, and the supernatant was poured out, the remaining solid was pulped with MTBE (15 mL, 15 vol.) at 15-25 °C for 2-3 hours, and filtered. The solid was washed with MTBE (15 mL, 15 vol.), and dried in vacuum to obtain 0.97g of crude Semaglutide as an off-white solid. The product was subjected to HPLC detection, and the HPLC chromatogram was shown in Figure 17. Liquid phase purity: 87.30%, molar yield: 93.50%.

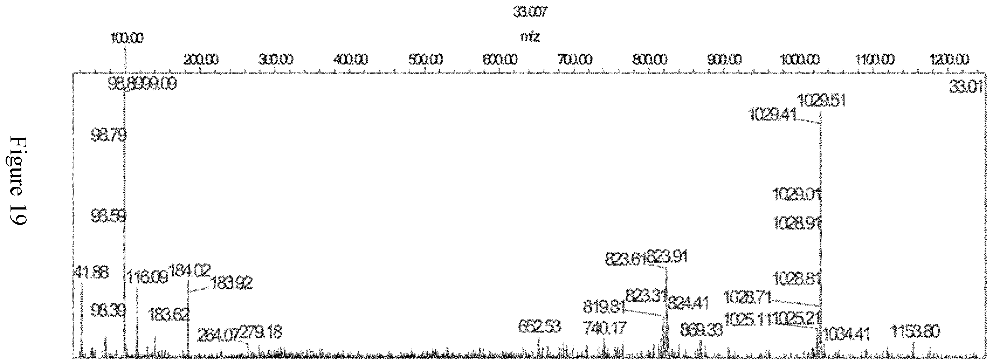

Mass spectrometry information: m/z=1029.71 (M+4H)⁴⁺. A typical mass spectrum of Semaglutide was shown in Figure 19.

### (2) Purification and separation of the crude product

The crude Semaglutide (0.50g, purity: 89.56%) was diluted in DMF/water 1:1 to a concentration of 50mg/mL and then subjected to a preparative column (column model RP18-OBD^{™}). Detection wavelength: 280nm, mobile phase A: 5mmol/L ammonium bicarbonate aqueous solution; mobile phase B: acetonitrile; and flow rate: 15 mL/min. Please refer to Table 2 for details.

**Table 2**

| Time/min | 0 | 15 | 16 | 18 | 19 | 25 |
|---|---|---|---|---|---|---|
| Mobile phase A (volume%) | 80 | 50 | 10 | 10 | 80 | 80 |
| Mobile phase B (volume%) | 20 | 50 | 90 | 90 | 20 | 20 |

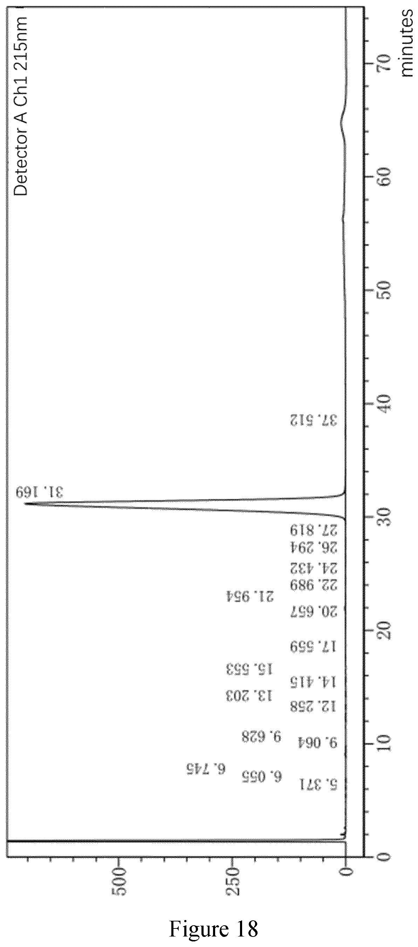

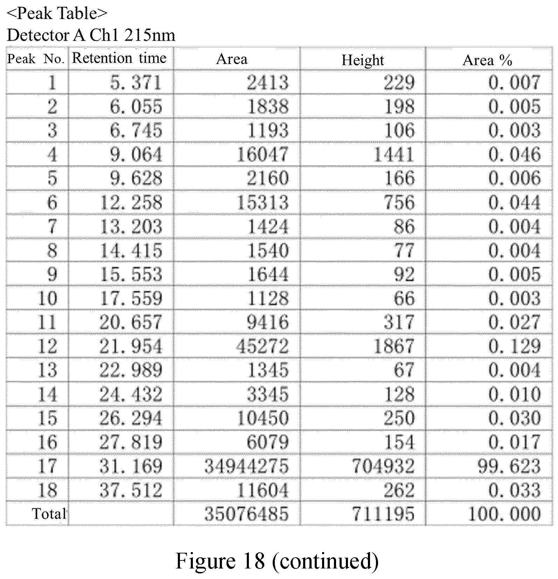

Product peaks were collected, acetic acid (13mg, 2.0eq.) was added and the resulting mixture was freeze-dried to obtain 390mg of Semaglutide as a white solid. The product was subjected to HPLC detection, and the HPLC chromatogram was shown in Figure 18. Liquid phase purity: 99.62%, molar yield: 88.02%.

Mass spectrometry information: m/z=1029.71 (M+4H)⁴⁺.

### Example 12: Preparation of Semaglutide

Semaglutide was prepared using compound SEM130 prepared in Example 7 as the raw material, and the preparation, separation, and purification processes were the same as those in Example 11.

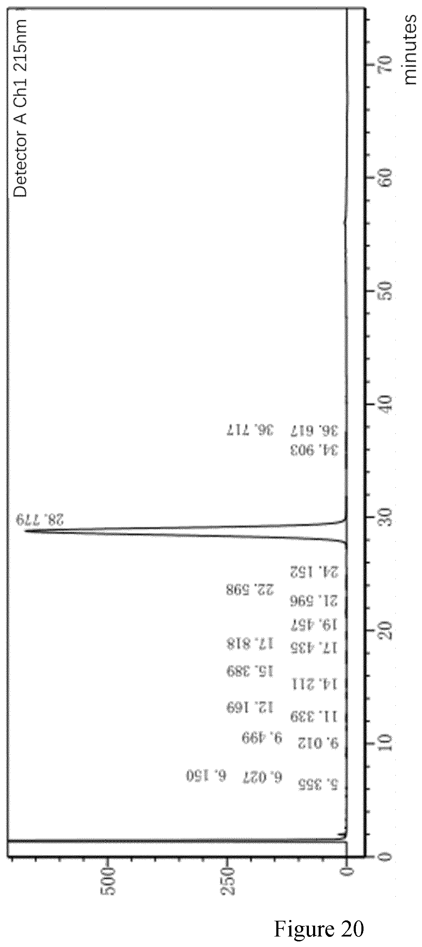

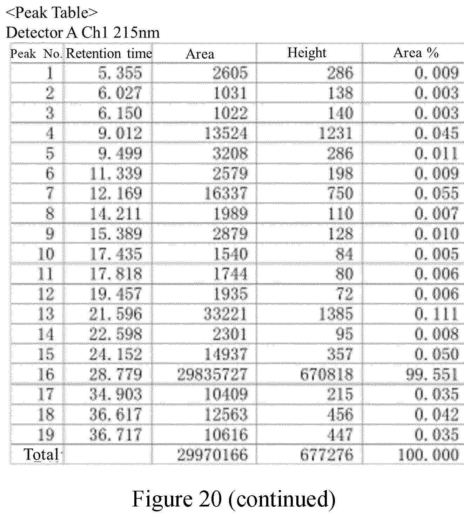

The product obtained after separation and purification was subjected to HPLC detection, and the HPLC chromatogram was shown in Figure 20. Liquid phase purity: 99.55%, molar yield: 84.30%.

Mass spectrometry information: m/z=1029.71 (M+4H)⁴⁺.

### Example 13: Preparation of Semaglutide

Semaglutide was prepared using compound SEM130 prepared in Example 8 as the raw material, and the preparation, separation, and purification processes were the same as those in Example 11.

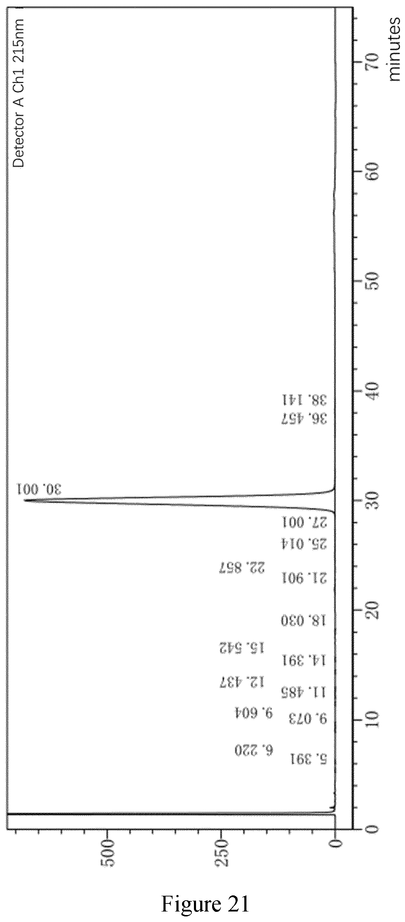

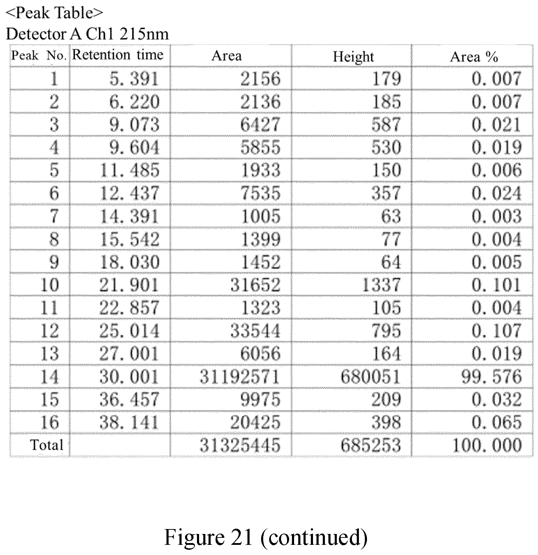

The product obtained after separation and purification was subjected to HPLC detection, and the HPLC chromatogram was shown in Figure 21. Liquid phase purity: 99.58%, molar yield: 82.58%.

Mass spectrometry information: m/z=1029.71 (M+4H)⁴⁺.

### Example 14: Preparation of Semaglutide

Semaglutide was prepared using compound SEM130 prepared in Example 10 as the raw material, and the preparation, separation, and purification processes were the same as those in Example 11.

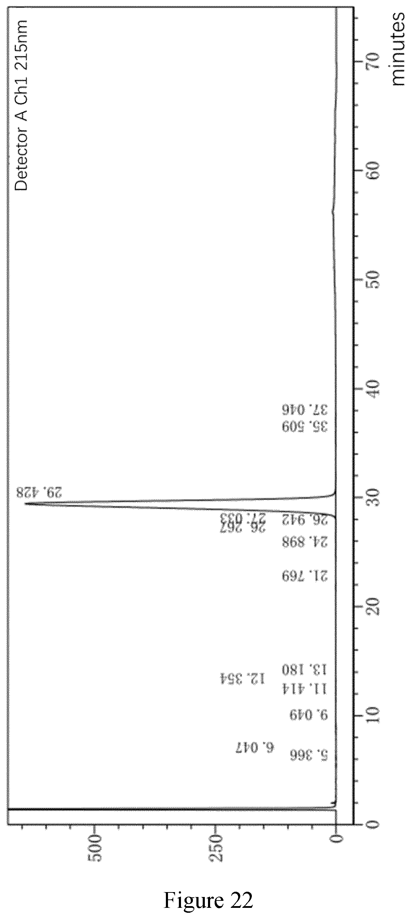

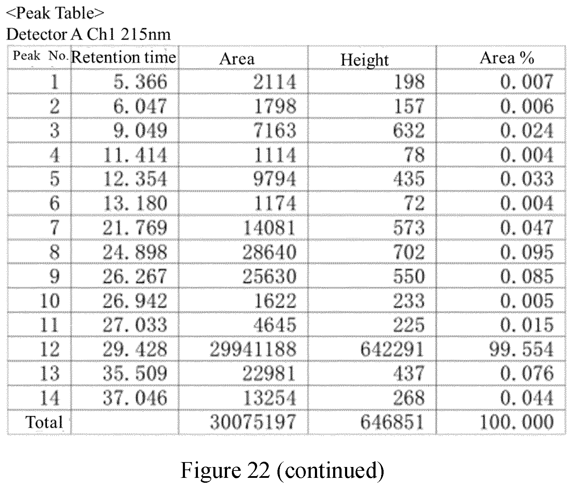

The product obtained after separation and purification was subjected to HPLC detection, and the HPLC chromatogram was shown in Figure 22.

Liquid phase purity: 99.55%, molar yield: 83.50%.

Mass spectrometry information: m/z=1029.71 (M+4H)⁴⁺.

### Example 15: Preparation of compound SEM100C using SEM80 and methylsulfonic anhydride

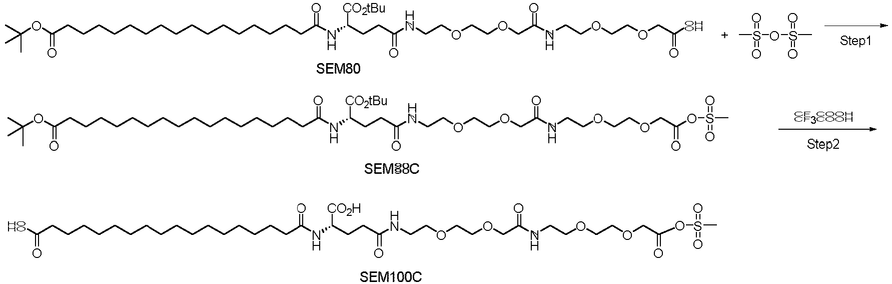

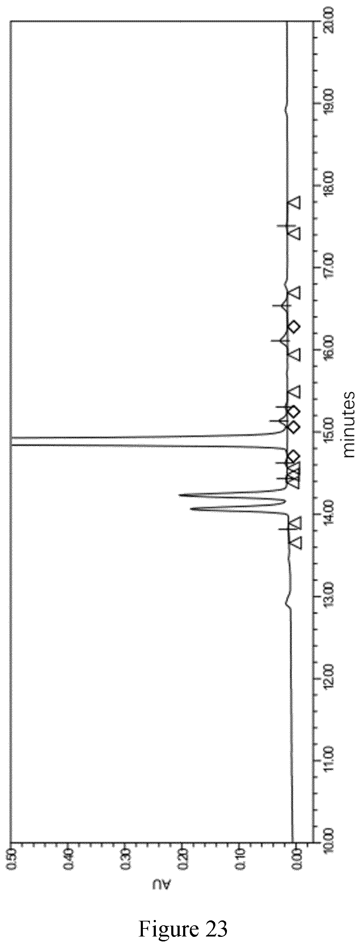

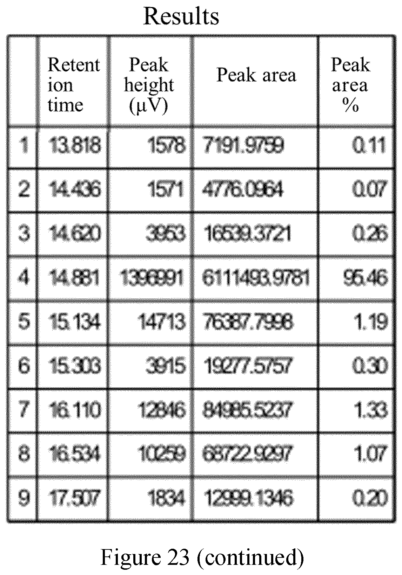

(1) Under nitrogen protection, the temperature was controlled at 15-30 °C. SEM80 (10 g, 11.82 mmol, 1.0 eq.), DCM (80 mL, 8.0 vol.), and methylsulfonic anhydride (2.16 g, 12.41 mmol, 1.05 eq.) were added in sequence and the mixture was stirred to dissolve. The resulting mixture was cooled to 0-10 °C in an ice water bath, DIPEA (1.83 g, 14.18 mmol, 1.2 eq.) was added, and after addition, the mixture was kept at 0-10 °C until the reaction was completed. The system was filtered and the filter cake was discarded. The filtrate was concentrated at 15-30 °C to 1.5-2.0 vol. to obtain a DCM solution of compound SEM90C.
(2) The DCM solution of compound SEM90C was cooled to 0-10 °C. The temperature was controlled at 0-10 °C, and DCM (10 mL, 1.0 vol.) and TFA (40 mL, 4.0 vol.) were added in sequence. After addition, the system was warmed to 10-20 °C and reacted until the conversion of SEM90C was complete. MTBE (200 mL, 20 vol.) was added dropwise at 10-20 °C. After stirring for 2 hours, the resulting mixture was filtered, and the filter cake was washed with MTBE (50 mL, 5 vol.) and vacuum-dried to obtain 12.26 g of SEM100C as an off-white solid. The product was an active ester, and the sample processing method was as follows: SEM100C was taken, quenched with excess cyclohexylamine, and subjected to HPLC and mass spectrometry detection. The HPLC spectrum was shown in Figure 23.

Mass spectrometry information: m/z=815.61 (M+H)⁺. A typical mass spectrum was shown in Figure 24.

### Example 16: Preparation of compound SEM100D using SEM80 andp-nitrophenol

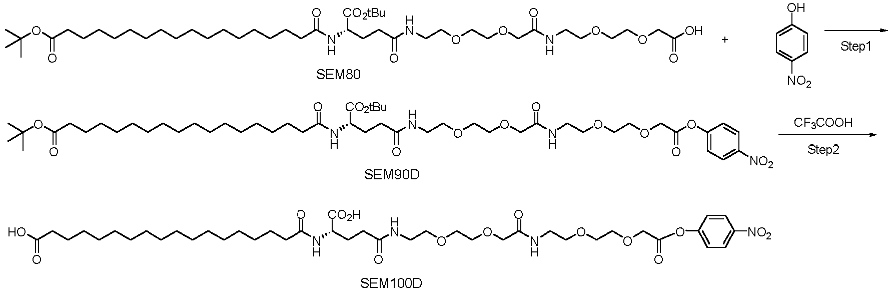

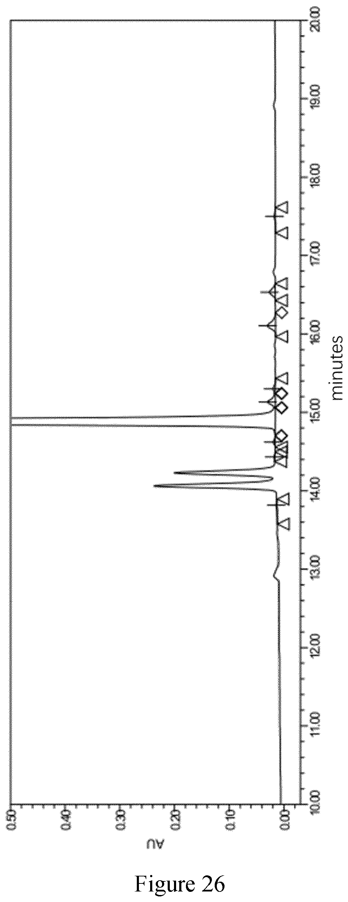

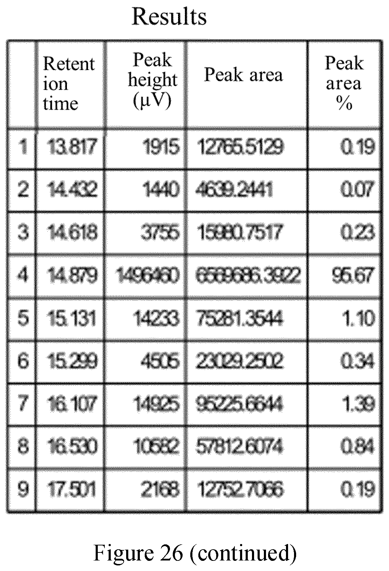

(1) Under nitrogen protection, the temperature was controlled at 15-30 °C. SEM80 (10 g, 11.82 mmol, 1.0 eq.), DCM (80 mL, 8.0 vol.), and p-nitrophenol (1.72 g, 12.41 mmol, 1.05 eq.) were added in sequence and the mixture was stirred to dissolve. The resulting mixture was cooled to 0-10 °C in an ice water bath, DCC (2.9 g, 14.18 mmol, 1.2 eq.) was added, and after addition, the mixture was kept at 0-10 °C until the reaction was completed. The system was filtered and the filter cake was discarded. The filtrate was concentrated at 15-30 °C to 1.5-2.0 vol. to obtain a DCM solution of compound SEM90D.
(2) The DCM solution of compound SEM90D was cooled to 0-10 °C. The temperature was controlled at 0-10 °C, and DCM (10 mL, 1.0 vol.) and TFA (40 mL, 4.0 vol.) were added in sequence. After addition, the system was warmed to 10-20 °C and reacted until the conversion of SEM90D was complete. MTBE (200 mL, 20 vol.) was added dropwise at 10-20 °C. After stirring for 2 hours, the resulting mixture was filtered. The filter cake was washed with MTBE (50 mL, 5 vol.) and vacuum-dried to obtain 13.10 g solid of SEM100D. The product was an active ester, and the sample processing method was as follows: SEM100D was taken, quenched with excess cyclohexylamine, and subjected to HPLC and mass spectrometry detection. The HPLC spectrum was shown in Figure 26. Liquid phase purity: 95.67%.

Mass spectrometry information: m/z=815.61 (M+H)⁺, a typical mass spectrum was shown in Figure 24.

### Example 17: Preparation of compound SEM130 using compound SEM100B, GLP-1 (9-37) peptide, and compound 120B

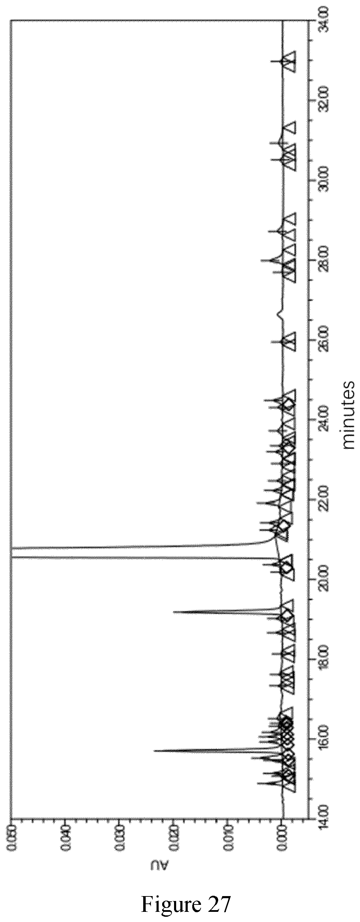

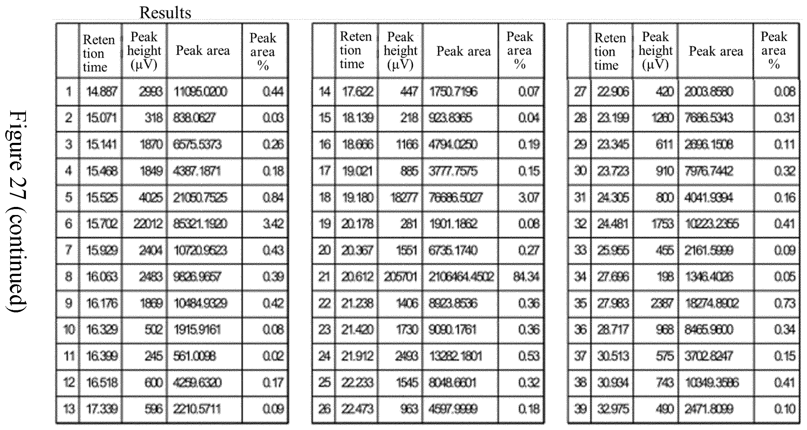

(1) The preparation process of compound SEM110 was the same as that in Example 7, except that the raw material SEM100 B was different;
(2) The anhydrous crude SEM110 (1.0g, 0.257 mmol, 1 eq.) was added to DMF (150 mL, 15 vol.), DIPEA was added to adjust the pH to 8-9, and the resulting mixture was stirred to dissolve. SEM120B (0.72g, 1.03mmol, 4 eq.) was added and the mixture was kept at 0-15 °C until the reaction was completed. Acetic acid was added dropwise to adjust the pH to 4-6. Acetonitrile (45 mL, 35 vol.) was added dropwise, and a large amount of solid precipitated. The resulting mixture was centrifuged to obtain crude SEM130, which was pulped with MTBE (150 mL, 15 vol.) at 15-25 °C for 2-3 hours, and filtered. The filter cake was dried in vacuum to obtain 1.35g of SEM130 as an off-white solid. The product was subjected to HPLC detection, and the HPLC chromatogram was shown in Figure 27. Liquid phase purity: 84.34%

Mass spectrometry information: m/z=1114.27 (M+4H) ⁴⁺.

### Example 18: Preparation of compound SEM130 using compound SEM100C, GLP-1 (9-37) peptide, and compound 120B

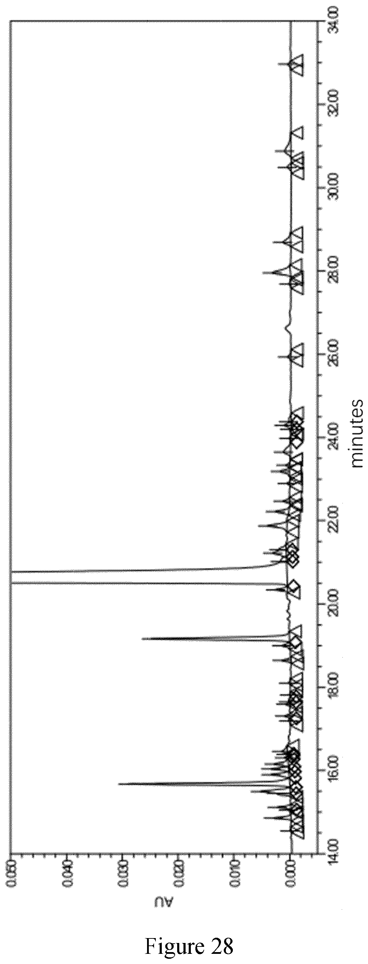

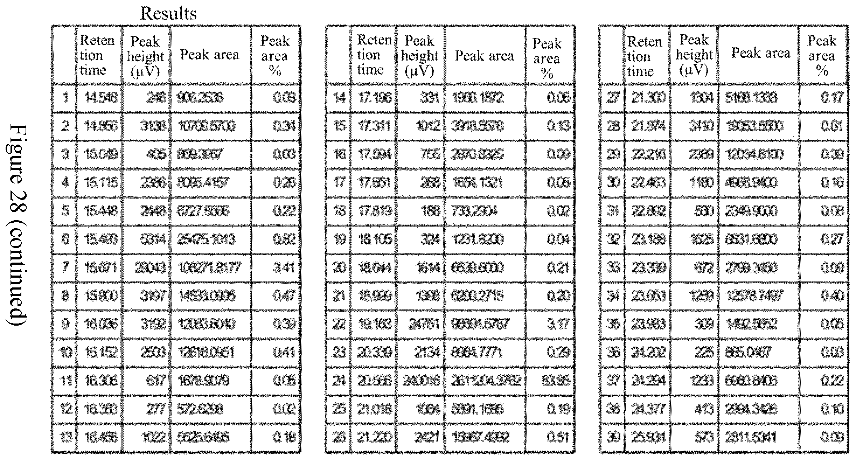

(1) The preparation process of compound SEM110 was the same as that in Example 7, except that the raw material SEM100C was different;
(2) The anhydrous crude SEM110 (1.0g, 0.257 mmol, 1 eq.) was added to DMF (150 mL, 15 vol.), DIPEA was added to adjust the pH to 8-9, and the resulting mixture was stirred to dissolve. SEM120B (0.72g, 1.03mmol, 4 eq.) was added and the mixture was kept at 0-15 °C until the reaction was completed. Acetic acid was added dropwise to adjust the pH to 4-6. Acetonitrile (45 mL, 35 vol.) was added dropwise, and a large amount of solid precipitated. The resulting mixture was centrifuged to obtain crude SEM130, which was pulped with MTBE (150 mL, 15 vol.) at 15-25 °C for 2-3 hours, and filtered. The filter cake was dried in vacuum to obtain 1.34g of SEM130 as an off-white solid. The product was subjected to HPLC detection, and the HPLC chromatogram was shown in Figure 28. Liquid phase purity: 83.86%

Mass spectrometry information: m/z=1114.27 (M+4H) ⁴⁺.

### Example 19: Preparation of compound SEM130 using compound SEM100D, GLP-1 (9-37) peptide, and compound 120B

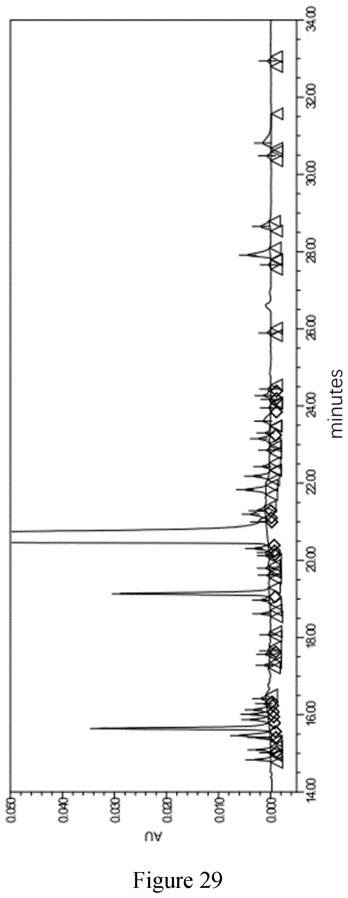

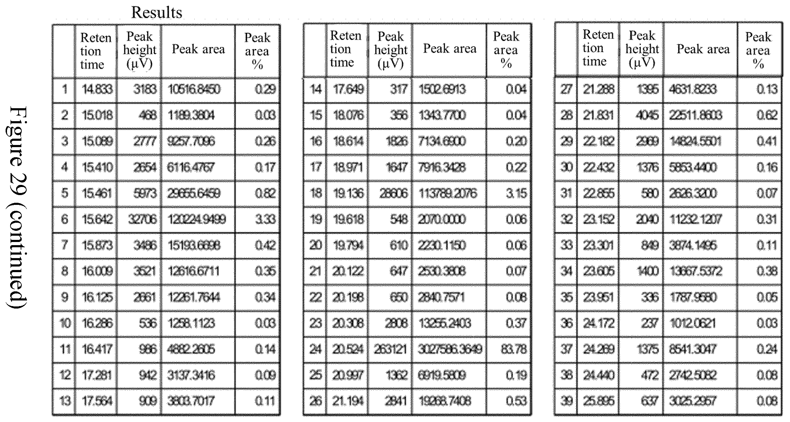

(1) The preparation process of compound SEM110 was the same as that in Example 7, except that the raw material SEM100D was different;
(2) The anhydrous crude SEM110 (1.0g, 0.257 mmol, 1 eq.) was added to DMF (150 mL, 15 vol.), DIPEA was added to adjust the pH to 8-9, and the resulting mixture was stirred to dissolve. SEM120B (0.72g, 1.03mmol, 4 eq.) was added and the mixture was kept at 0-15 °C until the reaction was completed. Acetic acid was added dropwise to adjust the pH to 4-6. Acetonitrile (45 mL, 35 vol.) was added dropwise, and a large amount of solid precipitated. The resulting mixture was centrifuged to obtain crude SEM130, which was pulped with MTBE (150 mL, 15 vol.) at 15-25 °C for 2-3 hours, and filtered. The filter cake was dried in vacuum to obtain 1.46g of SEM130 as an off-white solid. The product was subjected to HPLC detection, and the HPLC chromatogram was shown in Figure 29. Liquid phase purity: 83.78%

Mass spectrometry information: m/z=1114.27 (M+4H) ⁴⁺.

### Example 20: Preparation of Semaglutide

Semaglutide was prepared using compound SEM130 prepared in Example 17 as the raw material, and the preparation, separation, and purification processes were the same as those in Example 11.

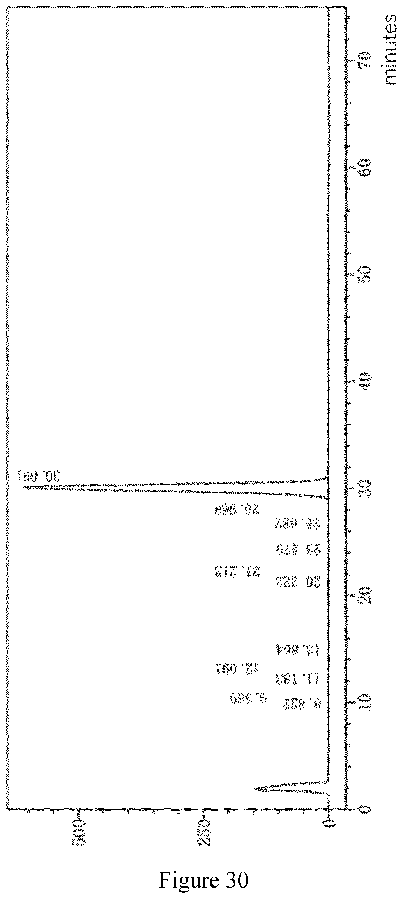

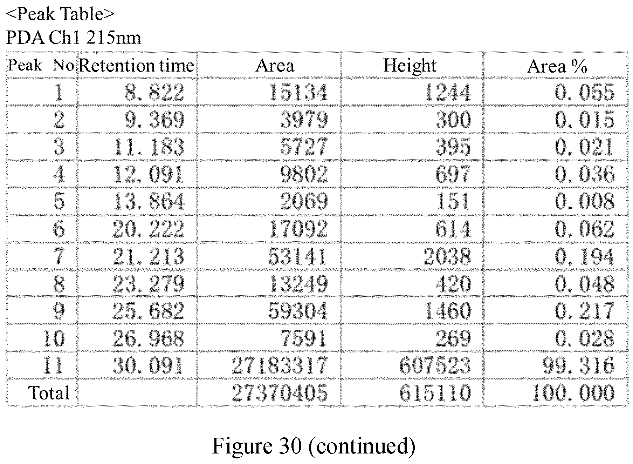

The product obtained after separation and purification was subjected to HPLC detection, and the HPLC chromatogram was shown in Figure 30. Liquid phase purity: 99.31%, molar yield: 81.50%.

### Example 21: Preparation of Semaglutide

Semaglutide was prepared using compound SEM130 prepared in Example 18 as the raw material, and the preparation, separation, and purification processes were the same as those in Example 11.

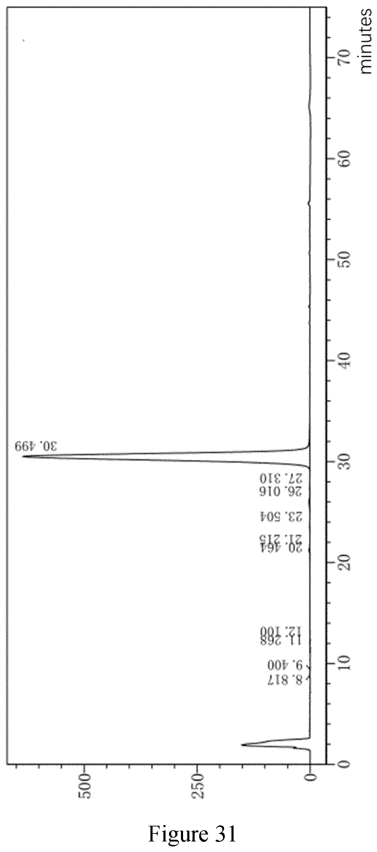

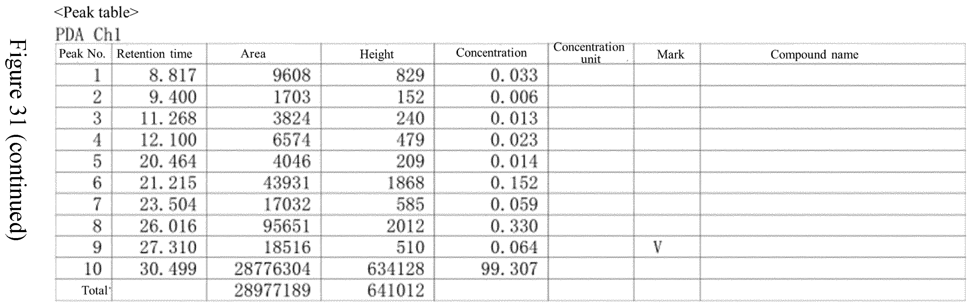

The product obtained after separation and purification was subjected to HPLC detection, and the HPLC chromatogram was shown in Figure 31. Liquid phase purity: 99.31%, molar yield: 80.72%.

### Example 22: Preparation of Semaglutide

Semaglutide was prepared using compound SEM130 prepared in Example 19 as the raw material, and the preparation, separation, and purification processes were the same as those in Example 11.

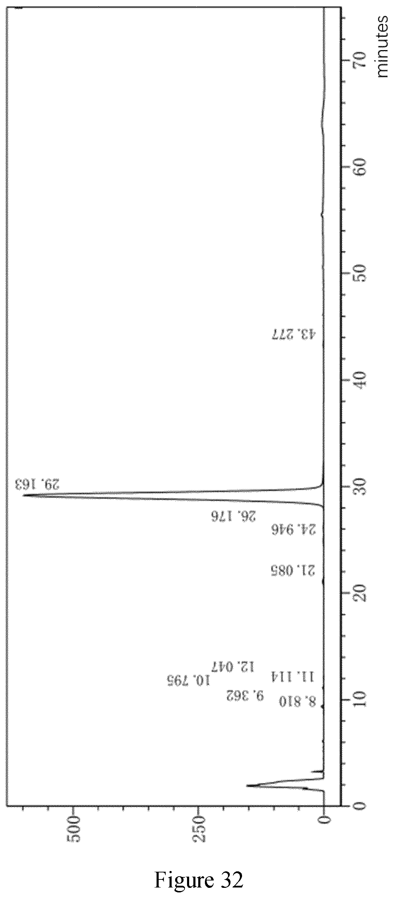

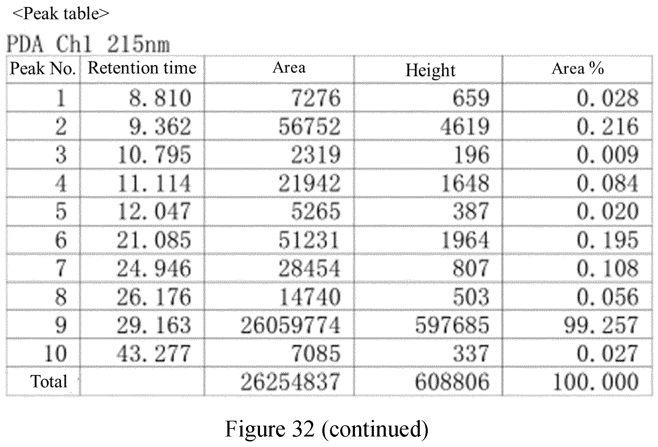

The product obtained after separation and purification was subjected to HPLC detection, and the HPLC chromatogram was shown in Figure 32. Liquid phase purity: 99.26%, molar yield: 82.71%.

### Comparative Example 1: Preparation of compound SEM100E using compound SEM80 and HOSU

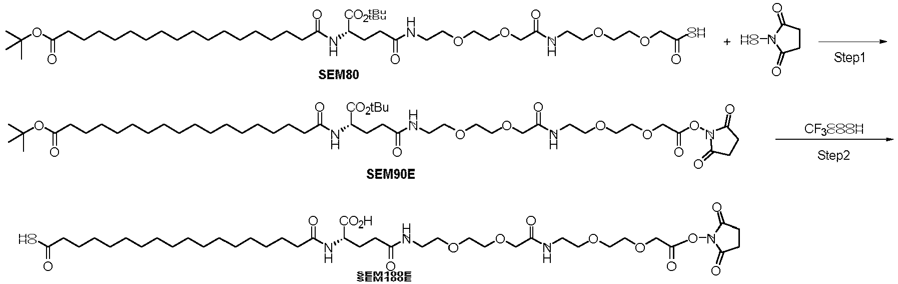

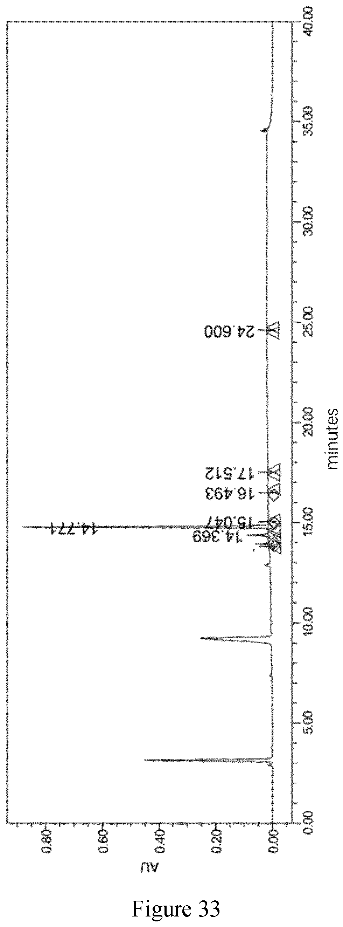

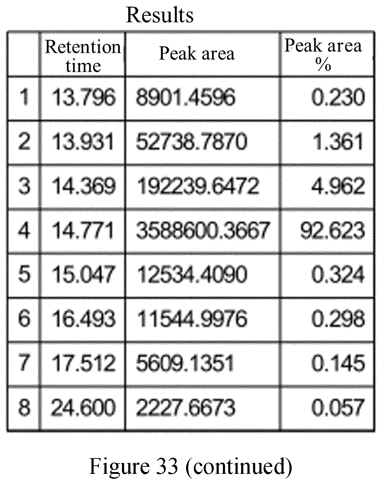

(1) Under nitrogen protection, the temperature was controlled at 15-30 °C. SEM80 (10 g, 11.82 mmol, 1.0 eq.), DCM (80 mL, 8.0 vol.), and HOSU (1.42 g, 12.41 mmol, 1.05 eq.) were added in sequence and the mixture was stirred to dissolve. The resulting mixture was cooled to 0-10 °C in an ice water bath, and DCC (2.9 g, 14.18 mmol, 1.2 eq.) was added. After addition, the mixture was kept at 0-10 °C until the reaction was completed. The system was filtered and the filter cake was discarded. The filtrate was concentrated at 15-30 °C to 1.5-2.0 vol. to obtain a DCM solution of compound SEM90E.
(2) The DCM solution of compound SEM90E was cooled to 0-10 °C. The temperature was controlled at 0-10 °C, and DCM (10 mL, 1.0 vol.) and TFA (40 mL, 4.0 vol.) were added in sequence. After addition, the system was warmed to 10-20 °C and reacted until the conversion of SEM90E was completed. MTBE (200 mL, 20 vol.) was added dropwise at 10-20 °C. After stirring for 2 hours, the resulting mixture was filtered. The filter cake was washed with MTBE (50 mL, 5 vol.) and dried in vacuum to obtain 11.31 g of SEM100E as a white solid. The product was subjected to HPLC detection. The HPLC spectrum was shown in Figure 33. Liquid phase purity: 92.62%, molar yield: 84.60%. The content of compound IMP1 was about 4.96%.

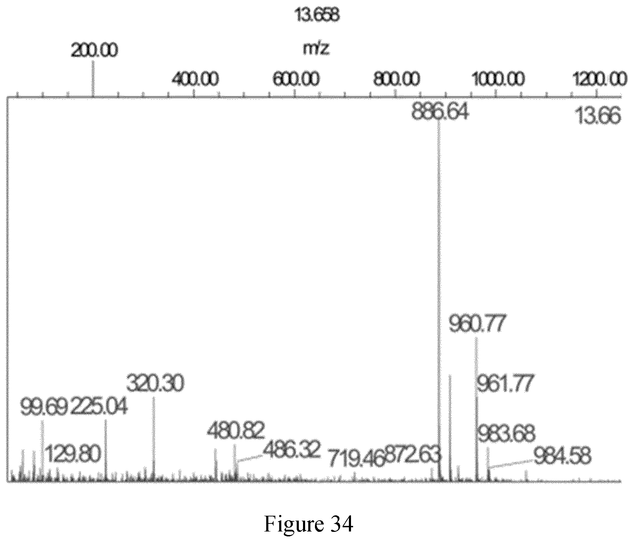

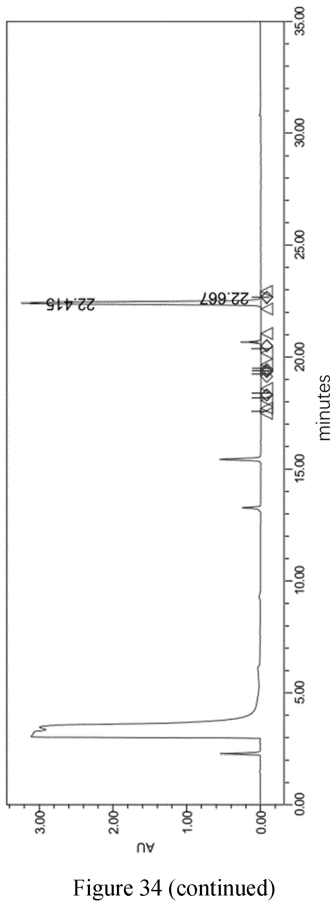

Mass spectrometry information of compound IMP1: m/z=886.64 (M+H)⁺. Mass spectrum was shown in Figure 34.

### Comparative Example 2: Preparation of compound SEM120E using compound SEM115 and HOSU

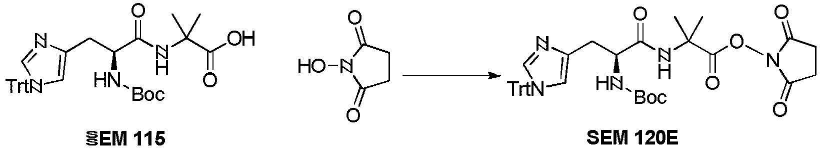

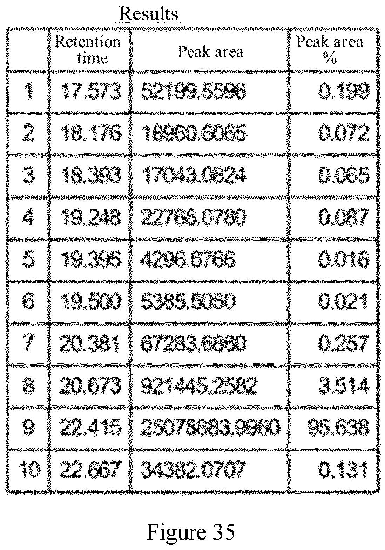

Under nitrogen protection, the temperature was controlled at 15-30 °C. Compound SEM115 (10 g, 17.16 mmol, 1.0 eq.), THF (80 mL, 8.0 vol.), and HOSU (2.07g, 18.02 mmol, 1.05 eq.) were added in sequence and the mixture was stirred to dissolve. The resulting mixture was cooled to 0-10 °C in an ice water bath, and DCC (4.25 g, 20.59 mmol, 1.2 eq.) was added. After addition, the mixture was kept at 0-10 °C until the reaction was completed. The system was filtered and the filter cake was discarded. The filtrate was concentrated at 15-30 °C to dryness to obtain 12.60 g of compound SEM120E as an off-white solid. The product was subjected to HPLC detection. The HPLC chromatogram was shown in Figure 35. Liquid phase purity: 95.64%, molar yield: 89.83%. The content of compound IMP2 was 3.51%.

The sample processing method was that the active ester system was quenched with excess water before sample detection.

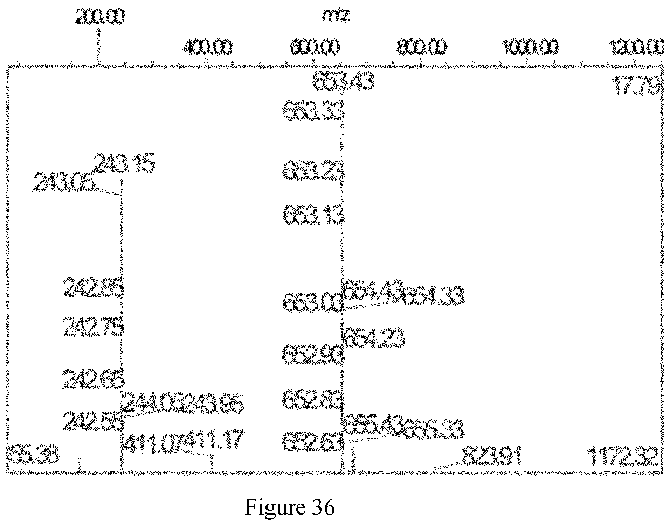

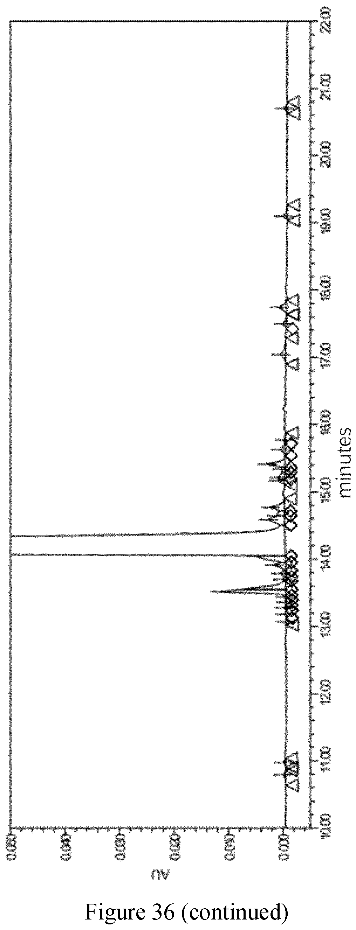

The mass spectrometry information of compound IMP2: m/z=653.43 (M+H)⁺. Please refer to Figure 36 for the mass spectrum.

### Comparative Example 3: Preparation of Semaglutide

The preparation of SEM130 was the same as that in Example 10 using compound SEM100E and compound SEM120A as raw materials, and the preparation, separation, and purification of SEM were the same as those in Example 11.

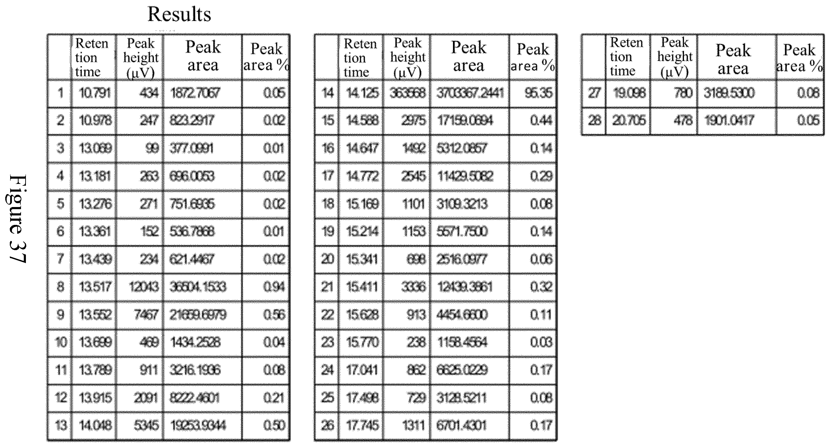

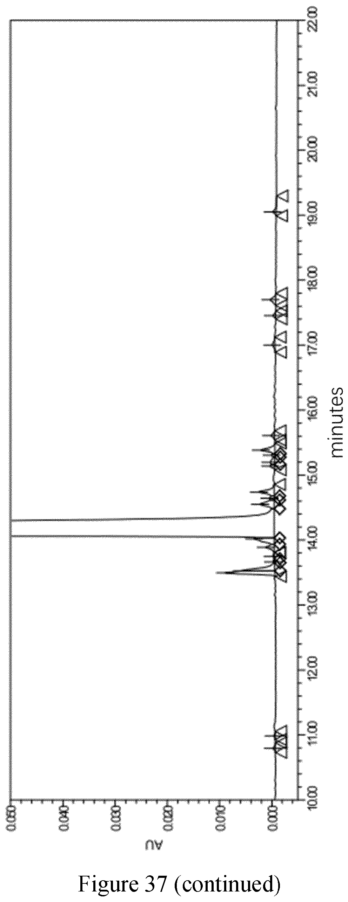

The product obtained after separation and purification was subjected to HPLC detection, and the HPLC spectrum was shown in Figure 37, with a liquid phase purity of 95.35% and a molar yield of 78.02%.

### Comparative Example 4: Preparation of Semaglutide

The preparation of SEM130 was the same as that in Example 10 using compounds SEM100A and SEM120E as raw materials,

The preparation, separation, and purification of SEM were the same as those in Example 11.

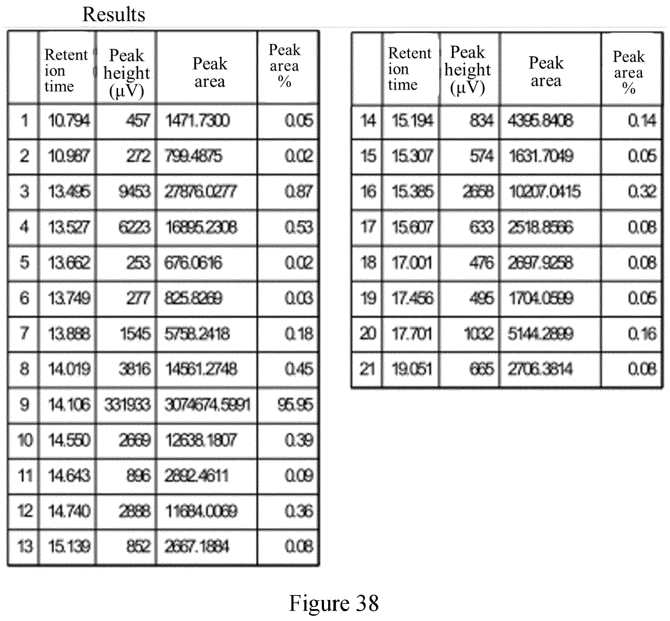

The product obtained after separation and purification was subjected to HPLC detection, and the HPLC chromatogram was shown in Figure 38.

Liquid phase purity: 95.95%, molar yield: 79.15%.

### Comparative Example 5: Preparation of Semaglutide

The preparation of SEM130 was the same as that in Example 10 using compounds SEM100E and SEM120E as raw materials, and the preparation, separation, and purification of SEM were the same as those in Example 11.

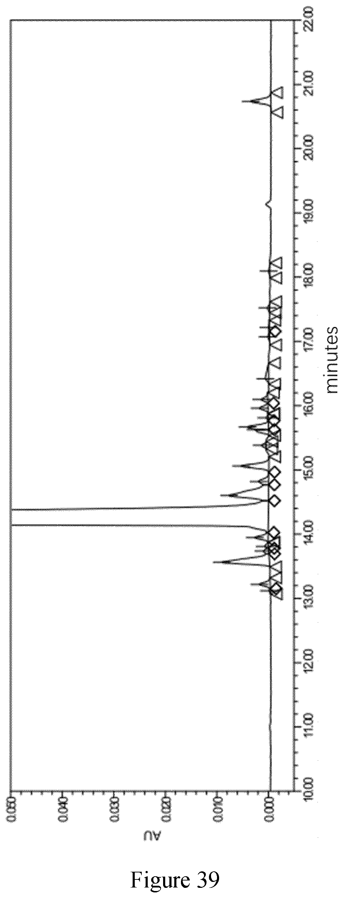

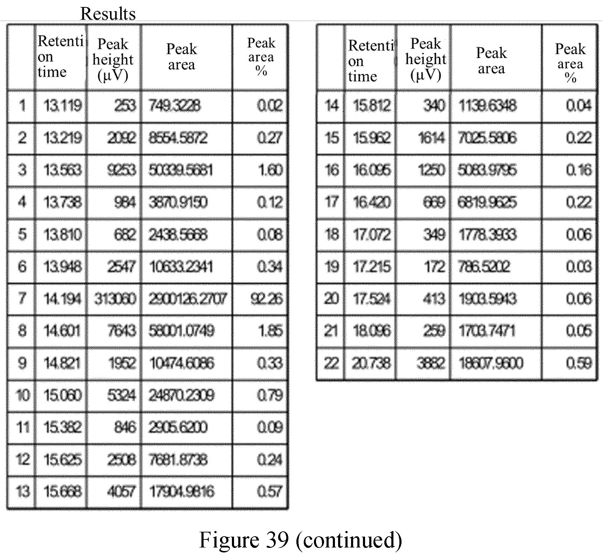

The product obtained after separation and purification was subjected to HPLC detection, and the HPLC chromatogram was shown in Figure 39, with a liquid phase purity of 92.26% and a molar yield of 78.02%.

Comparing the HPLC spectra of Semaglutide obtained from Comparative Examples 3, 4, and 5 with those obtained from Examples 11-14 and 20-22, it can be seen that using nitrophenol, methylsulfonic acid, pentafluorophenol, or HONB modified side chains and short chains according to the present invention avoids the impurities which is difficult to remove caused by the use of HOSU modified side chain and short chain.

In order to clearly demonstrate the impact of different combinations of activating groups on the final product, the data is summarized in Table 3 below.

**Table 3**

| Example | Side Chain SEM100 | Dipeptide SEM120 | Purified Semaglutide | Figure Number |
|---|---|---|---|---|
| Example 11 | SEM100A | SEM120A | 99.62 | 18 |
| Example 12 | SEM100A | SEM120B | 99.55 | 20 |
| Example 13 | SEM100A | SEM120C | 99.58 | 21 |
| Example 14 | SEM100A | SEM120D | 99.55 | 22 |
| Example 20 | SEM100B | SEM120B | 99.32 | 30 |
| Example 21 | SEM100C | SEM120B | 99.31 | 31 |
| Example 22 | SEM100D | SEM120B | 99.26 | 32 |
| Comparative Example 3 | SEM100E | SEM120A | 95.35 | 37 |
| Comparative Example 4 | SEM100A | SEM120E | 95.95 | 38 |
| Comparative Example 5 | SEM100E | SEM120E | 92.26 | 39 |

The above description is only preferred embodiments of the present invention and is not intended to limit the scope of the present invention. Various changes can also be made to the above embodiments of the present invention. That is, any simple, equivalent changes and modifications made based on the claims and specification of the present invention application fall within the protection scope of the claims of the present invention patent.

## Claims

1. A preparation method for semaglutide, wherein, the preparation method comprises the following steps of:
(1) reacting compound SEM110 and compound SEM120 in a solvent under an alkaline condition to obtain compound SEM130, and the reaction formula is as follows: 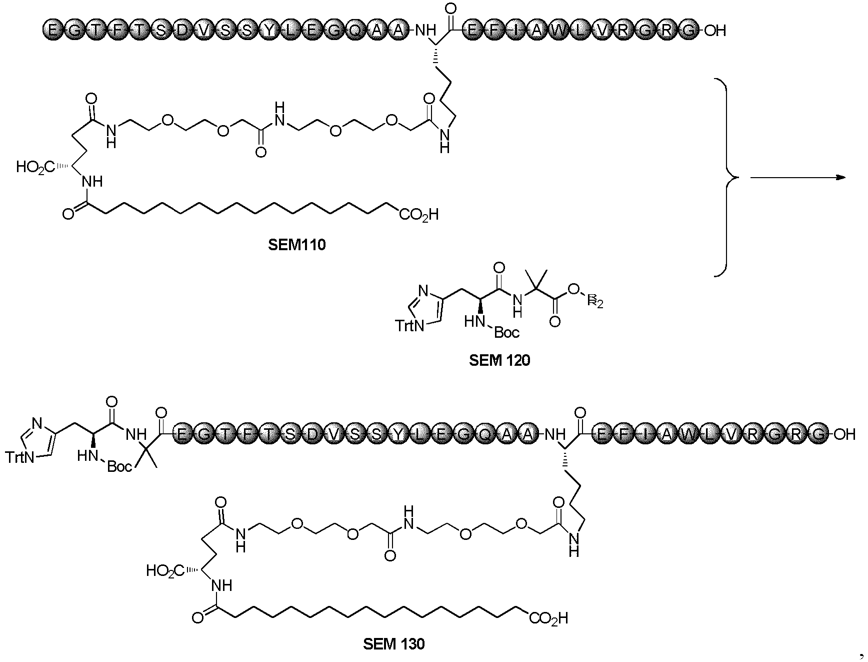 wherein, R₂ is *p*-nitrophenyl, methylsulfonyl, pentafluorophenyl or 5-norbornene-2,3-dicarboxamido;
(2) deprotecting the compound SEM130 to form semaglutide, and the reaction formula is as follows:

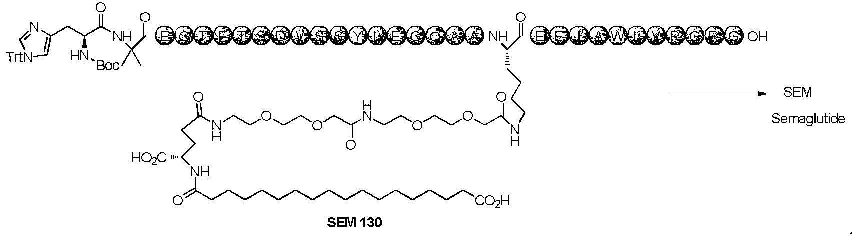

2. The preparation method according to claim 1, wherein R₂ is pentafluorophenyl.

3. The preparation method according to claim 1, wherein the step (1) possesses one or more features selected from the group consisting of:
the solvent is selected from one or more combinations of N, N-dimethylformamide, N-methylpyrrolidone, N, N-dimethylacetamide, tetrahydrofuran, dichloromethane, acetone, acetonitrile, and water;
the alkali used is selected from one or more combinations of carbonates, phosphates, bicarbonates, hydrophosphates, triethylamine, DBU, DMAP, and diisopropylethylamine, and more preferably diisopropylethylamine,
the molar ratio of compound SEM110 to compound SEM120 is 1:1.0-4.0, more preferably 1:2.0-3.0, and/or
the amount of alkali used is to maintain the pH of the system at 9-10, and more preferably 8-9.

4. The preparation method according to claim 1, wherein the step (2) possesses one or more features selected from the group consisting of:
the solvent used for the deprotection reaction is selected from one or more combinations of dichloromethane, methyl *tert*-butyl ether, trifluoroacetic acid, TIS, acetonitrile, and water,
deprotection is carried out in the presence of acid,
the acid is selected from one or more of TFA, acetic acid, hydrochloric acid, hydrobromic acid, phosphoric acid, and methylsulfonic acid, and more preferably TFA, acetic acid, and/or
the volume to weight ratio of the acid to compound SEM130 is 1-10 mL/g.

5. The preparation method according to claim 1, wherein the compound SEM120 is prepared by the following steps:
(1-d) reacting the compound SEM115 and compound 2 in a solvent in the presence of a dehydrating agent to form compound SEM120, and the reaction formula is as follows: 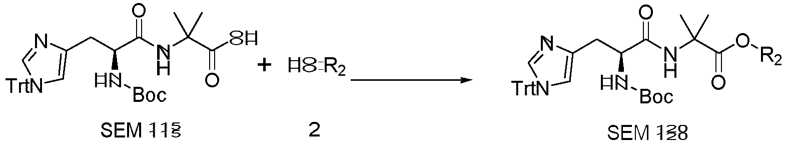
wherein, R₂ is *p*-nitrophenyl, methylsulfonyl, pentafluorophenyl or 5-norbornene-2,3-dicarboxamido.

6. The preparation method according to claim 5, wherein the step (1-d) possesses one or more features selected from the group consisting of:
the solvent is selected from one or more combinations of N, N-dimethylformamide, N-methylpyrrolidone, N, N-dimethylacetamide, tetrahydrofuran, dichloromethane, acetone, acetonitrile, and water,
the dehydrating agent is selected from one or more combinations of DCC, DIC, EDCI, and T₃P, and more preferably DCC,
the molar ratio of compound SEM115 to compound 2 is 1:1.0-1.4, more preferably 1:1.0-1.2, and/or
the molar ratio of dehydrating agent to compound SEM115 is 1.4-1.0:1, more preferably 1.3-1.1:1.

7. The preparation method according to claim 1, wherein the compound SEM110 is prepared by the following steps:
(1-c) reacting the compound SEM100 and compound SEM105 in a solvent in the presence of an alkali to form compound SEM110, and the reaction formula is as follows: 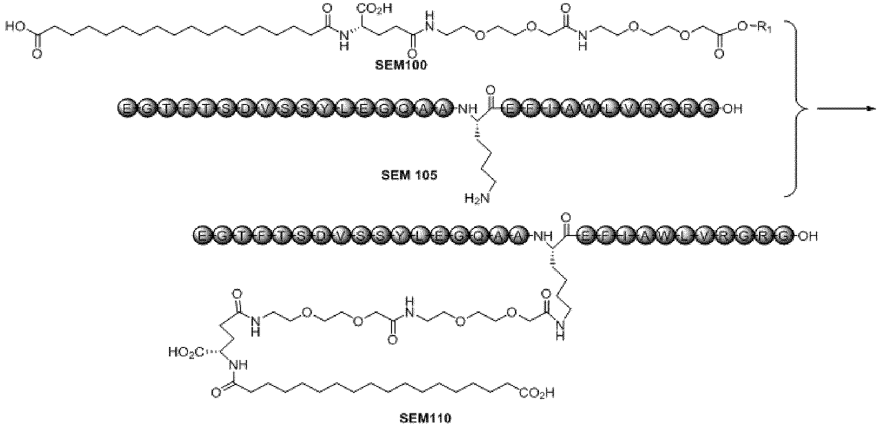
wherein, R₁ is *p*-nitrophenyl, methylsulfonyl, pentafluorophenyl or 5-norbornene-2,3-dicarboxamido.

8. The preparation method according to claim 7, wherein R₁ is 5-norbornene-2,3-dicarboxamido.

9. The preparation method according to claim 7, wherein the step (1-c) possesses one or more features selected from the group consisting of:
the solvent is selected from one or more combinations of N, N-dimethylformamide, N-methylpyrrolidone, N, N-dimethylacetamide, tetrahydrofuran, dichloromethane, acetone, acetonitrile, and water,
the alkali is selected from one or more combinations of carbonates, phosphates, bicarbonates, hydrophosphates, triethylamine, DBU, DMAP, and diisopropylethylamine, and more preferably diisopropylethylamine,
the molar ratio of compound SEM100 to compound SEM105 is 1.1-1.6:1, more preferably 1.2-1.5:1, and/or
the molar ratio of the alkali to compound SEM105 is 5-25:1 to compound SEM105, more preferably 10-20:1.

10. The preparation method according to claim 7, wherein the compound SEM100 is prepared by the following steps:
(1-a) reacting the compound SEM80 and compound 1 in a solvent in the presence of a dehydrating agent to form compound SEM90, and the reaction formula is as follows: 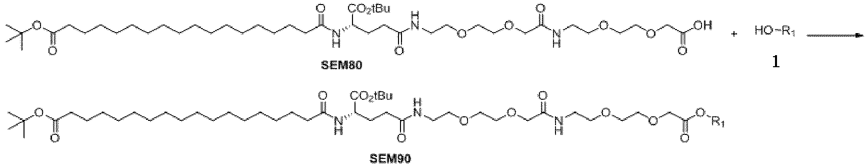 wherein, R₁ is *p*-nitrophenyl, methylsulfonyl, pentafluorophenyl or 5-norbornene-2,3-dicarboxamido;
(1-b) deprotecting compound SEM90 in a solvent in the presence of acid to form compound SEM100, and the reaction formula is as follows: 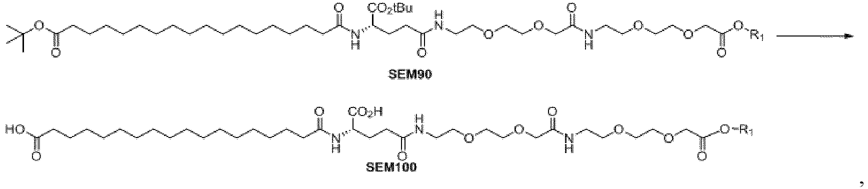 wherein, R₁ is *p*-nitrophenyl, methylsulfonyl, pentafluorophenyl or 5-norbornene-2,3-dicarboxamido.

11. The preparation method according to claim 10, wherein the step (1-a) possesses one or more features selected from the group consisting of:
the solvent is selected from one or more combinations of N, N-dimethylformamide, N-methylpyrrolidone, N, N-dimethylacetamide, tetrahydrofuran, dichloromethane, acetone, acetonitrile, and water,
the dehydrating agent in step (1-a) is selected from one or more combinations of DCC, DIC, EDCI, and T₃P, more preferably DCC,
the molar ratio of compound SEM80 to compound 1 is 1:1.0-1.4, more preferably 1:1.0-1.2, and most preferably 1:1.05, and/or
the molar ratio of compound SEM80 to the dehydrating agent is 1:1.0-1.4, more preferably 1:1.1-1.2.

12. The preparation method according to claim 10, wherein the step (1-b) possesses one or more features selected from the group consisting of:
the solvent in step (1-b) is selected from one or more combinations of dichloromethane, trifluoroacetic acid, TIS, acetonitrile, and water,
the acid is selected from one or more combinations of trifluoroacetic acid, acetic acid, hydrochloric acid, hydrobromic acid, phosphoric acid, and methylsulfonic acid, more preferably trifluoroacetic acid, acetic acid, and/or
the volume to weight ratio of the acid to compound 90 is 1-10 mL/g, more preferably 3-5 mL/g.

13. A compound SEM90 or the salts thereof, the structural formula of the compound is as follows: 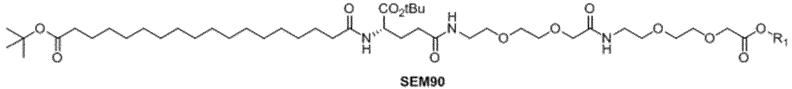 wherein, R₁ is methylsulfonyl or 5-norbornene-2,3-dicarboxamido.

14. A compound SEM100 or the salts thereof, the structural formula of the compound is as follows: 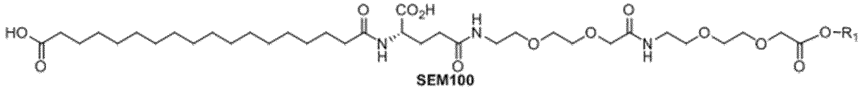 wherein, R₁ is *p*-methylsulfonyl, pentafluorophenyl or 5-norbornene-2,3-dicarboxamido.

15. A compound SEM120 or the salts thereof, the structural formula of the compound is as follows: 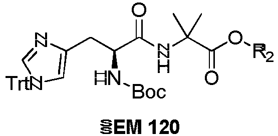 wherein, R₂ is methylsulfonyl, pentafluorophenyl or 5-norbornene-2,3-dicarboxamido.

16. A preparation method for the side chain of semaglutide, wherein the preparation method comprises the following steps:
(1-a) reacting the compound SEM80 and compound 1 in a solvent in the presence of a dehydrating agent to form compound SEM90, and the reaction formula is as follows: 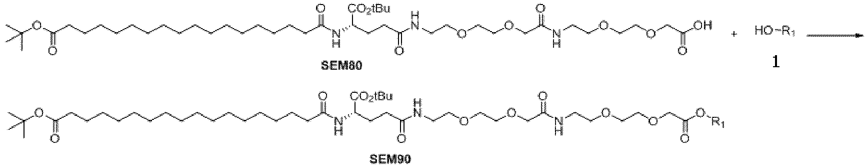 wherein, R₁ is *p*-nitrophenyl, methylsulfonyl, pentafluorophenyl or 5-norbornene-2,3-dicarboxamido;
(1-b) deprotecting compound SEM90 in a solvent in the presence of acid to form compound SEM100, and the reaction formula is as follows: 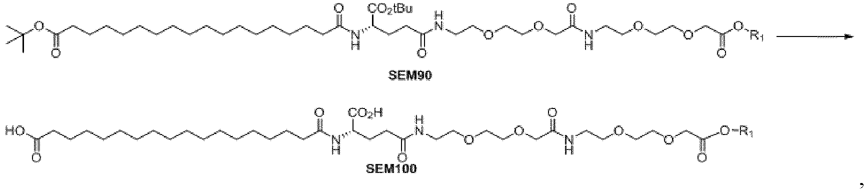 wherein, R₁ is *p*-nitrophenyl, methylsulfonyl, pentafluorophenyl or 5-norbornene-2,3-dicarboxamido.

17. A preparation method for an intermediate of semaglutide, wherein the preparation method comprises the following steps:
(1-a) reacting the compound SEM80 and compound 1 in a solvent in the presence of a dehydrating agent to form compound SEM90, and the reaction formula is as follows: 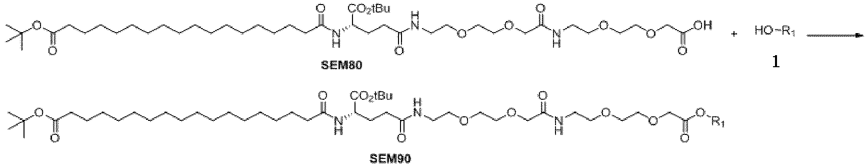 wherein, R₁ is p-nitrophenyl, methylsulfonyl, pentafluorophenyl or 5-norbornene-2,3-dicarboxamido;
(1-b) deprotecting compound SEM90 in a solvent in the presence of acid to form compound SEM100, and the reaction formula is as follows: 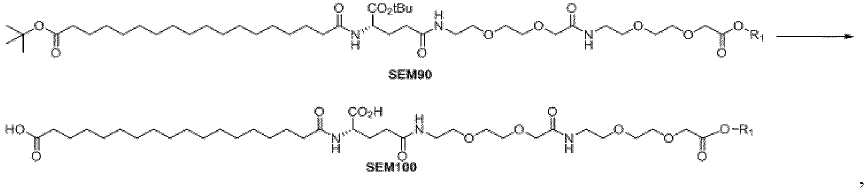 wherein, R₁ is p-nitrophenyl, methylsulfonyl, pentafluorophenyl or 5-norbornene-2,3-dicarboxamido;
(1-c) reacting the compound SEM100 and compound SEM105 in a solvent in the presence of an alkali to form compound SEM110, and the reaction formula is as follows: 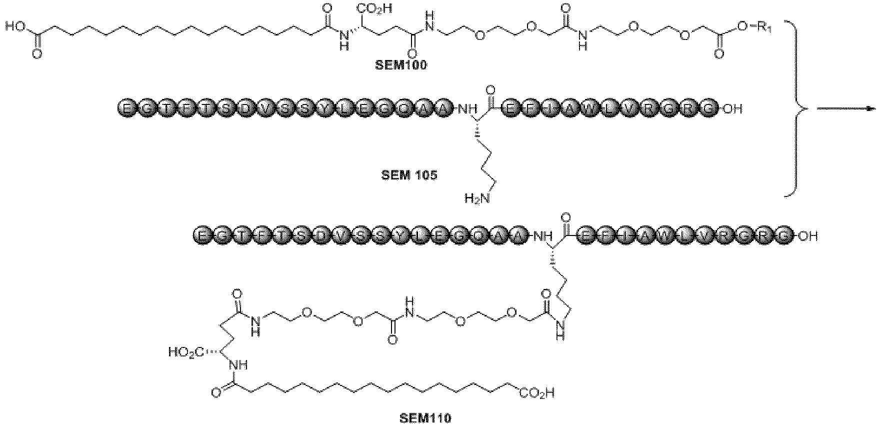 wherein, R₁ is p-nitrophenyl, methylsulfonyl, pentafluorophenyl or 5-norbornene-2,3-dicarboxamido.

18. A preparation method for semaglutide, comprising the following steps:
(1) reacting compound SEM110 and compound SEM120 in a solvent under an alkaline condition to obtain compound SEM130; and
(2) deprotecting the compound SEM130 to form semaglutide,
wherein, step (1) further includes the following steps:
(1-a) reacting the compound SEM80 and compound 1 in a solvent in the presence of a dehydrating agent to form SEM90;
(1-b) deprotecting compound SEM90 in a solvent in the presence of acid to form compound SEM100;
(1-c) reacting the compound SEM100 and compound SEM105 in a solvent in the presence of an alkali to form **SEM110;** and
(1-d) reacting the compound shown in SEM115 and compound 2 in a solvent in the presence of a dehydrating agent to form SEM120;
the reaction equation for the above steps is as follows: 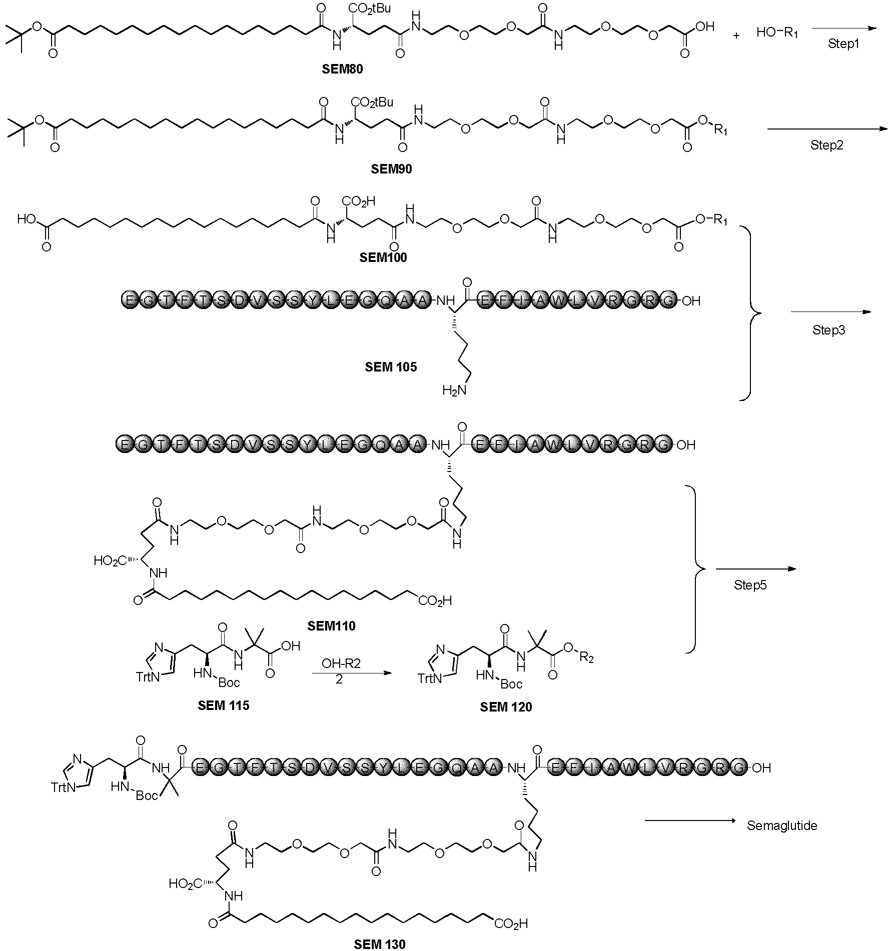
wherein, R₁ is *p*-nitrophenyl, methylsulfonyl, pentafluorophenyl or 5-norbornene-2,3-dicarboxamido, R₂ is *p*-nitrophenyl, methylsulfonyl, pentafluorophenyl or 5-norbornene-2,3-dicarboxamido, and
there is no order between steps (1-d) and steps (1-a) to (1-c), and they can be interchanged with any of them.
